# FASCICULE DE BREVET EUROPEEN

(11) **EP 3 519 589 B1**
(45) Date de publication et mention de la délivrance du brevet: **23.09.2020**
(21) Numéro de dépôt: 17784893.4
(22) Date de dépôt: 02.10.2017
(51) Int. Cl.: C12Q 1/68, C12Q 1/689

(54) **METHODE ET KIT DE DETECTION, DE DISCRIMINATION ET IDENTIFICATION D'ESPECES DE BORRELIES PRESENTES DANS UN ECHANTILLON D'ORIGINE HUMAINE OU ANIMALE**
VERFAHREN UND KIT ZUR DETECTION, DISCIMINIERUNG UND IDENTIFIZIERUNG VON BORRELIEN SPEZIEN IN EINE PROBE VON MENSCHLICHER ODER TIERISCHER URSPRUNG
METHOD AND KIT FOR THE DETECTION, DISCRIMINATION, IDENTIFICATION OF BORRELIA SPECIES PRESENT IN A SAMPLE OF HUMAN OR ANIMAL ORIGIN

(30) Priorité: 03.10.2016 FR 1659525
(43) Date de publication de la demande: 07.08.2019
(73) Titulaire: D.F. Conseils, 10190 Fontvannes (FR)
(72) Inventeur: AUVRAY, Pierrick, 35400 Saint Malo (FR); DESSAUGE, Elise, 35400 Saint Malo (FR); FRITZ, Denis, 10000 Troyes (FR)
(74) Mandataire: Cabinet Le Guen Maillet
(86) Numéro de dépôt international: PCT/EP2017/074988
(87) Numéro de publication internationale: WO 2018/065367

(56) Documents cités:
- WO-A2-91/19814
- WO-A2-2009/155103
- CN-A- 105 755 151
- CN-B- 103 255 168
- BJÖRN OLSEN ET AL: "A Lyme borreliosis cycle in seabirds and Ixodes uriae ticks", LETTERS TO NATURE, vol. 362, 25 mars 1993 (1993-03-25), pages 340-342, XP055386115,
- ANDRIAS HOJGAARD ET AL: "Detection of Borrelia burgdorferi, Anaplasma phagocytophilum and Babesia microti, with two different multiplex PCR assays", TICKS AND TICK-BORNE DISEASES, vol. 5, no. 3, 1 avril 2014 (2014-04-01), pages 349-351, XP055386121, AMSTERDAM, NL ISSN: 1877-959X, DOI: 10.1016/j.ttbdis.2013.12.001
- HAITHAM ELBIR ET AL: "Multiplex Real-Time PCR Diagnostic of Relapsing Fevers in Africa", PLOS NEGLECTED TROPICAL DISEASES, vol. 7, no. 1, 31 janvier 2013 (2013-01-31), page e2042, XP055386124, DOI: 10.1371/journal.pntd.0002042
- NUNES MÓNICA ET AL: "Molecular identification ofBorreliagenus in questing hard ticks from Portugal: Phylogenetic characterization of two novel Relapsing Fever-likeBorreliasp", INFECTION, GENETICS AND EVOLUTION, vol. 40, 11 mars 2016 (2016-03-11), pages 266-274, XP029523015, ISSN: 1567-1348, DOI: 10.1016/J.MEEGID.2016.03.008

### SEQUENCE LISTING

<110> CAL
<120> méthode et kit de détection et de discrimination des espèces de borrélies présentes dans un échantillon humain ou animal.
<130> 22316
<160> 37
<170> BiSSAP 1.3.6
<210> 1
   <211> 19
   <212> DNA
   <213> Artificial Sequence
<220>
   <223> Borrelia 16S RNA forward primer A
<400> 1
   cgttgttcgg gattattgg
   19
<210> 2
   <211> 21
   <212> DNA
   <213> Artificial Sequence
<220>
   <223> Borrelia16S RNA reverse primerA
<400> 2
   cagattccac ccttacacca g
   21
<210> 3
   <211> 13
   <212> DNA
   <213> Artificia1 Sequence
<220>
   <223> Borrelia lyme 16S RNA probe
<400> 3
   atagaggaag tta
   13
<210> 4
   <211> 14
   <212> DNA
   <213> Artificial Sequence
<220>
   <223> Borrelia feaver 16S RNA probe
<400> 4
   catgactaga gtct
   14
<210> 5
   <211> 20
   <212> DNA
   <213> Artificial Sequence
<220>
   <223> actine forward primer
<400> 5
   acccacactg tgcccatcta
   20
<210> 6
   <211> 20
   <212> DNA
   <213> Artificial Sequence
<220>
   <223> Actine reverse primer
<400> 6
   cttgatgtca cgcacgattt
   20
<210> 7
   <211> 20
   <212> DNA
   <213> Artificial Sequence
<220>
   <223> Actine probe
<400> 7 <210> 8
   <211> 20
   <212> DNA
   <213> Artificial Sequence
<220>
   <223> Borrelia flagelline forward primer B
<400> 8
<210> 9
   <211> 23
   <212> DNA
   <213> Artificial Sequence
<220>
   <223> Borrelia falgelline reverse primer B 1
<400> 9
   acaggagaat taactccacc ytg
   23
<210> 10
   <211> 21
   <212> DNA
   <213> Artificial Sequence
<220>
   <223> Borrelia falgelline reverse prime rB 2
<400> 10
   aggagaatta actccgcctt g
   21
<210> 11
   <211> 27
   <212> DNA
   <213> Artificial Sequence
<220>
   <223> B. burgdorferi sensu stricto flagelline probe
<400> 11
   tcaagagggt gttcaacagg aaggagc
   27
<210> 12
   <211> 16
   <212> DNA
   <213> Artificial Sequence
<220>
   <223> B.afzelii flagelline probe
<400> 12
   agggtgctca agaaga
   16
<210> 13
   <211> 31
   <212> DNA
   <213> Artificial Sequence
<220>
   <223> B. Garinii flagelline probe
<400> 13
   caggctgctc agactgcacc tgttcaagaa g
   31
<210> 14
   <211> 24
   <212> DNA
   <213> Artificial Sequence
<220>
   <223> Borrelia flagelline forward primer C 1
<400> 14
   accaagatga agctattgct gtaa
   24
<210> 15
   <211> 21
   <212> DNA
   <213> Artificial Sequence
<220>
   <223> Borrelia flagelline forward primer C 2
<400> 15
   aagatgaggc gattgctgta a
   21
<210> 16
   <211> 19
   <212> DNA
   <213> Artificial Sequence
<220>
   <223> Borrellia flagelline reverse primer C
<400> 16
   caggtgctgg ytgttgagc
   19
<210> 17
   <211> 17
   <212> DNA
   <213> Artificial Sequence
<220>
   <223> B. Valaisiana flagelline probe
<400> 17
   ctacacctgt tcaagaa
   17
<210> 18
   <211> 17
   <212> DNA
   <213> Artificial Sequence
<220>
   <223> B. Bissentii flagelline probe
<400> 18
   tcgcaaatct tttctct
   17
<210> 19
   <211> 21
   <212> DNA
   <213> Artificial Sequence
<220>
   <223> Borrelia flagelline forward primer D
<400> 19
   aaatcttttt gcaggtgaag g
   21
<210> 20
   <211> 21
   <212> DNA
   <213> Artificial Sequence
<220>
   <223> Borellia Falgelline reverse primer D
<400> 20
   catcaacagc ggttgtaaca t
   21
<210> 21
   <211> 21
   <212> DNA
   <213> Artificial Sequence
<220> <223> Borrealia 16S RNA forward primer
<400> 21
   ggaaatgaca aggtgatgac g
   21
<210> 22
   <211> 23
   <212> DNA
   <213> Artificial Sequence
<220>
   <223> Borrelia 16S RNA reverse primer
<400> 22
   gcagtttcta gcatagctcc aca
   23
<210> 23
   <211> 16
   <212> DNA
   <213> Artificial Sequence
<220>
   <223> B. Hermsii Flagelline probe
<400> 23
   aagagatagg acagca
   16
<210> 24
   <211> 14
   <212> DNA
   <213> Artificial Sequence
<220>
   <223> B. recurrentis 16S RNA probe
<400> 24
   agtcccggct aatt
   14
<210> 25
   <211> 21
   <212> DNA
   <213> Artificial Sequence
<220>
   <223> Borrelia Rec A forward primer E
<400> 25
   gatggcgaga tgggtgatac t
   21
<210> 26
   <211> 25
   <212> DNA
   <213> Artificial Sequence
<220>
   <223> Borrelia Rec A reverse primer E
<400> 26
   gcatttcctc cagtagtagt ctctg
   25
<210> 27
   <211> 13
   <212> DNA
   <213> Artificial Sequence
<220>
   <223> B. duttonii et B. crocidurae RecA probe
<400> 27
   agccatactt tca
   13
<210> 28
   <211> 153
   <212> DNA
   <213> Borrelia
<220>
   <223> Borrelia Lyme
<400> 28
   cgttgttcgg gattattggg cgtaaagggt gagtaggcgg atatataagt ctatgcataa
   60
   aataccacag ctcaactgtg gaactatgtt ggaaactata tgtctagagt ctgatagagg
   120
   aagttagaat ttctggtgta agggtggaat ctg
   153
<210> 29
   <211> 152
   <212> DNA
   <213> Borrelia
<220>
   <223> Borrelia Feaver
<400> 29
   cgttgttcgg gattattggg cgtaaagggt gagtaggcgg atatgcaagt ctatgcgtaa 60
   aataccacag ctcaactgtg gaactagctg gaaactgcat gactagagtc tgatagggga 120
   agttagaatt cctggtgtaa gggtggaatc tg 152
<210> 30
   <211> 182
   <212> DNA
   <213> Borrelia burgdorferi
<400> 30
   ggagcaaacc aagatgaagc tattgctgta aatatttatg cagctaatgt tgcaaatctt 60
   ttctctggtg agggagctca aactgctcag gctgcaccgg ttcaagaggg tgttcaacag 120
   gaaggagctc aacagccagc acctgctaca gcaccttctc aaggcggagt taattctcct 180
   gt 182
<210> 31
   <211> 180
   <212> DNA
   <213> Borrelia afzelii
<400> 31
   ggagcaaatc aagatgaagc aattgctgta aatatttatt cagctaatgt tgcaaatctt 60
   tttgctggtg agggagctca agctgctcag gctgcacctg ttcaagaggg tgctcaagaa 120
   gaaggagctc agcaaccaac acctgctaca gcacctactc aaggtggagt taattctcct 180
<210> 32
   <211> 179
   <212> DNA
   <213> Borrelia garinii
<400> 32
   ggagcaaatc aagatgaagc gattgctgta aatatttatg ctctaatgtt gcaaatctat 60
   tctctggtga aggagctcag gctgctcaga ctgcacctgt tcaagaaggt gctcaacaag 120
   aaggagctca acaaccagca cctgctacag cgccttctca gggtggagtt aattctcct 179
<210> 33
   <211> 134
   <212> DNA
   <213> Borrelia valaisiana
<400> 33
   aagatgaggc gattgctgta aatatttatg cagctaatgt tgcaaatctg ttttctggtg 60
   agggagctca aactgctcag gctaacctgt tcaagaaggt gctcaacagg aaggagctca 120
   acaaccagca cctg 134
<210> 34
   <211> 138
   <212> DNA
   <213> Borrelia bissettii
<400> 34
   accaagatga agctattgct gtaaatattt atgcagctaa tgtcgcaaat cttttctctg 60
   gtgagggagc tcaaactgct caggctgcac ctgttcaaga aggtgttcag caagaaggag 120
   ctcaacagcc agcacctg 138
<210> 35
   <211> 146
   <212> DNA
   <213> Borrelia hermsii
<400> 35
   aaatcttttt gcaggtgaag gcgctcaggc tgctccagtg caagagatag gacagcaaga 60
   ggaaggtcaa gcagctccag ctccagcagc agctccagct caaggtggag ttaattcccc 120
   aattaatgtt acaaccgctg ttgatg 146
<210> 36
   <211> 184
   <212> DNA
   <213> Borrelia recurrentis
<400> 36
   ggaaatgaca aggtgatgac gttaatttat gaataagtcc cggctaatta cgtgccagca 60
   gccgcggtaa tacgtaaggg gcgagcgttg ttcgggatta ttgggcgtaa agggtgagta 120
   ggcggatatg caagtctgtg cgtaaaatac cacagctcaa ctgtggagct atgctagaaa 180
   ctgc 184
<210> 37
   <211> 176
   <212> DNA
   <213> Borrelia crocidurae
<220>
   <223> Borrelia crocidurae et duttonii
<400> 37
   gatggcgaga tgggtgatac tcaaattgga cttcaagcaa gacttatgag taaggcttta 60
   aggaaaatta cagccatact ttcaaaatct aatacttgta ttatgtttat taatcaaata 120
   agaatgaaaa ttggtttagt ctttggtagt ccagagacta ctactggagg aaatgc 176

### SEQUENCE LISTING

<110> CAL
<120> methode et kit de detection et de discrimination des especes de
   Borrelies présentes dans un échantillon humain ou animal.
<130> 22316
<160> 37
<170> BiSSAP 1.3.6
<210> 1
   <211> 19
   <212> DNA
   <213> Artificial Sequence
<220>
   <223> Borrelia 16S RNA forward primer A
<400> 1
   cgttgttcgg gattattgg 19
<210> 2
   <211> 21
   <212> DNA
   <213> Artificial Sequence
<220>
   <223> Borrelia16S RNA reverse primerA
<400> 2
   cagattccac ccttacacca g 21
<210> 3
   <211> 13
   <212> DNA
   <213> Artificial Sequence
<220>
   <223> Borrelia lyme 16S RNA probe
<400> 3
   atagaggaag tta 13
<210> 4
   <211> 14
   <212> DNA
   <213> Artificial Sequence
<220>
   <223> Borrelia feaver 16S RNA probe
<400> 4
   catgactaga gtct 14
<210> 5
   <211> 20
   <212> DNA
   <213> Artificial Sequence
<220>
   <223> actine forward primer
<400> 5
   acccacactg tgcccatcta 20
<210> 6
   <211> 20
   <212> DNA
   <213> Artificial Sequence
<220>
   <223> Actine reverse primer
<400> 6
   cttgatgtca cgcacgattt 20
<210> 7
   <211> 20
   <212> DNA
   <213> Artificial Sequence
<220>
   <223> Actine probe
<400> 7
   ggctacagct tcaccaccac 20
<210> 8
   <211> 20
   <212> DNA
   <213> Artificial Sequence
<220>
   <223> Borrelia flagelline forward primer B
<400> 8
   ggagcaaatc aagatgargc 20
<210> 9
   <211> 23
   <212> DNA
   <213> Artificial Sequence
<220>
   <223> Borrelia falgelline reverse primer B 1
<400> 9
   acaggagaat taactccacc ytg 23
<210> 10
   <211> 21
   <212> DNA
   <213> Artificial Sequence
<220>
   <223> Borrelia falgelline reverse prime rB 2
<400> 10
   aggagaatta actccgcctt g 21
<210> 11
   <211> 27
   <212> DNA
   <213> Artificial Sequence
<220>
   <223> B. burgdorferi sensu stricto flagelline probe
<400> 11
   tcaagagggt gttcaacagg aaggagc 27
<210> 12
   <211> 16
   <212> DNA
   <213> Artificial Sequence
<220>
   <223> B.afzelii flagelline probe
<400> 12
   agggtgctca agaaga 16
<210> 13
   <211> 31
   <212> DNA
   <213> Artificial Sequence
<220>
   <223> B. Garinii flagelline probe
<400> 13
   caggctgctc agactgcacc tgttcaagaa g 31
<210> 14
   <211> 24
   <212> DNA
   <213> Artificial Sequence
<220>
   <223> Borrelia flagelline forward primer C 1
<400> 14
   accaagatga agctattgct gtaa 24
<210> 15
   <211> 21
   <212> DNA
   <213> Artificial Sequence
<220>
   <223> Borrelia flagelline forward primer C 2
<400> 15
   aagatgaggc gattgctgta a 21
<210> 16
   <211> 19
   <212> DNA
   <213> Artificial Sequence
<220>
   <223> Borrellia flagelline reverse primer C
<400> 16
   caggtgctgg ytgttgagc 19
<210> 17
   <211> 17
   <212> DNA
   <213> Artificial Sequence
<220>
   <223> B. Valaisiana flagelline probe
<400> 17
   ctacacctgt tcaagaa 17
<210> 18
   <211> 17
   <212> DNA
   <213> Artificial Sequence
<220>
   <223> B. Bissentii flagelline probe
<400> 18
   tcgcaaatct tttctct 17
<210> 19
   <211> 21
   <212> DNA
   <213> Artificial Sequence
<220>
   <223> Borrelia flagelline forward primer D
<400> 19
   aaatcttttt gcaggtgaag g 21
<210> 20
   <211> 21
   <212> DNA
   <213> Artificial Sequence
<220>
   <223> Borellia Falgelline reverse primer D
<400> 20
   catcaacagc ggttgtaaca t 21
<210> 21
   <211> 21
   <212> DNA
   <213> Artificial Sequence
<220>
   <223> Borrealia 16S RNA forward primer
<400> 21
   ggaaatgaca aggtgatgac g 21
<210> 22
   <211> 23
   <212> DNA
   <213> Artificial Sequence
<220>
   <223> Borrelia 16S RNA reverse primer
<400> 22
   gcagtttcta gcatagctcc aca 23
<210> 23
   <211> 16
   <212> DNA
   <213> Artificial Sequence
<220>
   <223> B. Hermsii Flagelline probe
<400> 23
   aagagatagg acagca 16
<210> 24
   <211> 14
   <212> DNA
   <213> Artificial Sequence
<220>
   <223> B. recurrentis 16S RNA probe
<400> 24
   agtcccggct aatt 14
<210> 25
   <211> 21
   <212> DNA
   <213> Artificial Sequence
<220>
   <223> Borrelia Rec A forward primer E
<400> 25
   gatggcgaga tgggtgatac t 21
<210> 26
   <211> 25
   <212> DNA
   <213> Artificial Sequence
<220>
   <223> Borrelia Rec A reverse primer E
<400> 26
   gcatttcctc cagtagtagt ctctg 25
<210> 27
   <211> 13
   <212> DNA
   <213> Artificial Sequence
<220>
   <223> B. duttonii et B. crocidurae RecA probe
<400> 27
   agccatactt tca 13
<210> 28
   <211> 153
   <212> DNA
   <213> Borrelia
<220>
   <223> Borrelia Lyme
<400> 28
   cgttgttcgg gattattggg cgtaaagggt gagtaggcgg atatataagt ctatgcataa 60
   aataccacag ctcaactgtg gaactatgtt ggaaactata tgtctagagt ctgatagagg 120
   aagttagaat ttctggtgta agggtggaat ctg 153
<210> 29
   <211> 152
   <212> DNA
   <213> Borrelia
<220>
   <223> Borrelia Feaver
<400> 29
   cgttgttcgg gattattggg cgtaaagggt gagtaggcgg atatgcaagt ctatgcgtaa 60
   aataccacag ctcaactgtg gaactagctg gaaactgcat gactagagtc tgatagggga 120
   agttagaatt cctggtgtaa gggtggaatc tg 152
<210> 30
   <211> 182
   <212> DNA
   <213> Borrelia burgdorferi
<400> 30
   ggagcaaacc aagatgaagc tattgctgta aatatttatg cagctaatgt tgcaaatctt 60
   ttctctggtg agggagctca aactgctcag gctgcaccgg ttcaagaggg tgttcaacag 120
   gaaggagctc aacagccagc acctgctaca gcaccttctc aaggcggagt taattctcct 180
   gt 182
<210> 31
   <211> 180
   <212> DNA
   <213> Borrelia afzelii
<400> 31
   ggagcaaatc aagatgaagc aattgctgta aatatttatt cagctaatgt tgcaaatctt 60
   tttgctggtg agggagctca agctgctcag gctgcacctg ttcaagaggg tgctcaagaa 120
   gaaggagctc agcaaccaac acctgctaca gcacctactc aaggtggagt taattctcct 180
<210> 32
   <211> 179
   <212> DNA
   <213> Borrelia garinii
<400> 32
   ggagcaaatc aagatgaagc gattgctgta aatatttatg ctctaatgtt gcaaatctat 60
   tctctggtga aggagctcag gctgctcaga ctgcacctgt tcaagaaggt gctcaacaag 120
   aaggagctca acaaccagca cctgctacag cgccttctca gggtggagtt aattctcct 179
<210> 33
   <211> 134
   <212> DNA
   <213> Borrelia valaisiana
<400> 33
   aagatgaggc gattgctgta aatatttatg cagctaatgt tgcaaatctg ttttctggtg 60
   agggagctca aactgctcag gctaacctgt tcaagaaggt gctcaacagg aaggagctca 120
   acaaccagca cctg 134
<210> 34
   <211> 138
   <212> DNA
   <213> Borrelia bissettii
<400> 34
   accaagatga agctattgct gtaaatattt atgcagctaa tgtcgcaaat cttttctctg     60
   gtgagggagc tcaaactgct caggctgcac ctgttcaaga aggtgttcag caagaaggag    120
   ctcaacagcc agcacctg                                                  138
<210> 35
   <211> 146
   <212> DNA
   <213> Borrelia hermsii
<400> 35
   aaatcttttt gcaggtgaag gcgctcaggc tgctccagtg caagagatag gacagcaaga     60
   ggaaggtcaa gcagctccag ctccagcagc agctccagct caaggtggag ttaattcccc    120
   aattaatgtt acaaccgctg ttgatg                                         146
<210> 36
   <211> 184
   <212> DNA
   <213> Borrelia recurrentis
<400> 36
   ggaaatgaca aggtgatgac gttaatttat gaataagtcc cggctaatta cgtgccagca     60
   gccgcggtaa tacgtaaggg gcgagcgttg ttcgggatta ttgggcgtaa agggtgagta    120
   ggcggatatg caagtctgtg cgtaaaatac cacagctcaa ctgtggagct atgctagaaa    180
   ctgc                                                                 184
<210> 37
   <211> 176
   <212> DNA
   <213> Borrelia crocidurae
<220>
   <223> Borrelia crocidurae et duttonii
<400> 37
   gatggcgaga tgggtgatac tcaaattgga cttcaagcaa gacttatgag taaggcttta     60
   aggaaaatta cagccatact ttcaaaatct aatacttgta ttatgtttat taatcaaata    120
   agaatgaaaa ttggtttagt ctttggtagt ccagagacta ctactggagg aaatgc        176

## Description

La présente invention concerne une méthode de détection, de discrimination et identification d'espèce de borrélies présentes dans un échantillon, tel qu'un tissu ou un fluide, d'origine humaine ou animale, ainsi qu'un kit pour la mise en œuvre d'une telle méthode.

La borrélie est un genre de bactéries appartenant à la famille des spirochètes et comprenant plusieurs espèces que l'on peut classer en deux groupes en fonction de la pathologie qu'elles provoquent : un groupe responsable de la maladie de Lyme et un groupe responsable de fièvres récurrentes.

La maladie de Lyme est notamment transmise à l'homme ou l'animal suite à une morsure de tique infectée par des borrélies. Les bactéries vont alors se retrouver dans la salive de la tique et peuvent atteindre la circulation sanguine. Les symptômes de la maladie de Lyme sont une rougeur cutanée au niveau de la morsure évoluant en dehors de tout traitement vers des symptômes tels qu'un état grippal, une atteinte neurologique, une fatigue intense ou encore des troubles du rythme cardiaque. Parmi les borrélies responsables de la maladie de Lyme, on recense les espèces suivantes : B. burgdorferi sensu stricto, B. garinii, B. afzelii, B. spielmanii, B. valaisiana, B. bissettii, B. americana, B. andersonii, B. carolinensis, B. japonica, B. lusitaniae, B. sinica, B. tanukii et B. turdi.

Les fièvres récurrentes sont des infections bactériennes transmises par l'intermédiaire de morsures de poux et de tiques. Les fièvres peuvent être très intenses et provoquer des symptômes sévères tels que des frissons, des douleurs, des complications pulmonaires, voire des hémorragies cérébrales. Parmi les borrélies responsables de fièvres récurrentes, on recense les espèces suivantes : B. anserina, B. crocidurae, B. duttonii, B. hermsii, B. hispanica, B. miyamotoi, B. parkeri, B. persica, B. recurrentis, B. turicatae, B. lonestari, B. microti et B. theileri.

Les traitements des pathologies induites par les borrélies reposent essentiellement sur la prise d'antibiotiques adaptés.

De nombreux tests de détection des borrélies sont décrits dans la littérature mais beaucoup sont basés sur la technique de « polymorphisme de longueur des fragments de restriction », sur la détection d'anticorps par tests ELISA généralement confirmés par Western Blot ou par des méthodes d'amplification de séquences cibles d'ADN. Cependant, les tests aujourd'hui disponibles sont soit peu fiables avec un nombre de faux négatifs important, ne concernent que la détection de certaines espèces ou encore ne sont pas suffisamment discriminants pour distinguer des bactéries étroitement apparentées. Par ailleurs, la détection d'anticorps ne permet pas de distinguer entre une contamination ancienne ou une contamination en cours, les IgG pouvant persister plusieurs années après la guérison.

Enfin, les tests aujourd'hui proposés se montrent peu sensibles et sont ainsi à l'origine de résultats faussement négatifs. Or, de l'efficacité de la méthode de détection des borrélies et de l'identification des espèces présentes dans un échantillon contaminé, ainsi que de la sensibilité de la méthode, vont dépendre la rapidité de la mise en place du traitement ainsi que la spécificité du traitement en fonction de l'espèce identifiée.

Le but de la présente invention est ainsi de proposer une méthode de détection de borrélies, dans un échantillon d'origine humaine ou animale, qui soit sensible, efficace, reproductible, spécifique des borrélies et qui permette en outre la discrimination des espèces de borrélies entre elles, la méthode étant facile à mettre en œuvre.

A cet effet, l'invention concerne une méthode de détection de la présence de Borrélies dans un échantillon humain ou animal, de discrimination et d'identification d'espèces de borrélies, par PCR multiplexes quantitatives, comprenant :
(a) une étape de détection de la présence de borrélies dans l'échantillon et de distinction desdites borrélies entre le groupe provoquant la maladie de Lyme et le groupe provoquant des fièvres récurrentes, ladite étape consistant en :
   - l'extraction d'ADN dudit échantillon,
   - l'amplification de séquences nucléotidiques cibles dudit ADN à l'aide d'une paire d'amorces incluant l'amorce sens de séquence SEQ ID NO :1 et l'amorce antisens de séquence SEQ ID NO :2 et de deux sondes consistant en les séquences SEQ ID NO :3 et SEQ ID NO :4, dans des conditions permettant la production d'amplicons, et,
   - la détection de la présence ou l'absence de borrélies du groupe provoquant la maladie de Lyme ou du groupe provoquant des fièvres récurrentes par détection de la présence d'un signal de fluorescence résultant de la formation d'amplicons, puis,
   si un signal est détecté à l'étape (a) indiquant la présence de borrélies du groupe de la maladie de Lyme,
(b) une étape de détection et de distinction, dans l'échantillon, de la présence des espèces B. burgdorferi sensu stricto, B. garinii ou B. afzelii et des espèces B. valaisiana ou B. bissettii, ladite étape consistant en :
   - à partir de l'ADN extrait dudit échantillon,
   - (b1) l'amplification de séquences nucléotidiques cibles dudit ADN à l'aide de paires d'amorces formées par l'amorce sens de séquence SEQ ID NO :8 et les amorces antisens de séquences SEQ ID NO :9 et SEQ ID NO :10 et de trois sondes consistant en les séquences SEQ ID NO :11, SEQ ID NO :12 et SEQ ID NO :13, dans des conditions permettant la production d'amplicons, et,
   - (b2) la détection de la présence ou l'absence de borrélies appartenant aux espèces B. burgdorferi sensu stricto, B. garinii ou B. afzelii par détection de la présence d'un signal de fluorescence résultant de la formation d'amplicons, et
   - (b3) l'amplification de séquences nucléotidiques cibles dudit ADN à l'aide de paires d'amorces formées par l'amorce sens de séquence SEQ ID NO :14, l'amorce sens de séquence SEQ ID NO :15 et l'amorce antisens de séquence SEQ ID NO :16, et de deux sondes consistant en les séquences SEQ ID NO :17 et SEQ ID NO :18, dans des conditions permettant la production d'amplicons, et,
   - (b4) la détection de la présence ou l'absence de borrélies appartenant aux des espèces B. valaisiana ou B. bissettii par détection de la présence d'un signal de fluorescence résultant de la formation d'amplicons,
   ou, si un signal est détecté à l'étape (a) indiquant la présence de borrélies du groupe des fièvres récurrentes,
(c) une étape de détection et de distinction, dans l'échantillon, de la présence des espèces B. hermsii et B. recurrentis, et des espèces B. duttonii ou B. crocidurae du groupe provoquant les fièvres récurrentes, ladite étape consistant en :
   - à partir de l'ADN extrait dudit échantillon,
   - (c1) l'amplification de séquences nucléotidiques cibles dudit ADN à l'aide d'une paire d'amorces incluant l'amorce sens de séquence SEQ ID NO :19 et l'amorce antisens de séquence SEQ ID NO :20 et d'une paire d'amorces incluant l'amorce sens de séquence SEQ ID NO :21 et l'amorce antisens de séquence SEQ ID NO :22, et de deux sondes consistant en les séquences SEQ ID NO :23 et SEQ ID NO :24, dans des conditions permettant la production d'amplicons, et
   - (c2) la détection de la présence ou l'absence de borrélies appartenant aux espèces B. hermsii et B. recurrentis, par détection de la présence d'un signal de fluorescence résultant de la formation d'amplicons, et,
   - (c3) l'amplification de séquences nucléotidiques cibles dudit ADN à l'aide d'une paire d'amorces incluant l'amorce sens de séquence SEQ ID NO :25 et l'amorce antisens de séquence SEQ ID NO :26 et d'une sonde consistant en la séquence SEQ ID NO :27, dans des conditions permettant la production d'amplicons, et,
   - (c4) la détection de la présence ou l'absence de borrélies appartenant aux espèces B. duttonii ou B. crocidurae par détection de la présence d'un signal de fluorescence résultant de la formation d'amplicons,
(d) l'analyse des résultats des amplifications,
étant entendu que ladite méthode inclut les séquences présentant au moins 90% d'homologie avec les séquences SEQ ID NO : 1 à 4 et 8 à 27.

La méthode selon l'invention permet de détecter les borrélies présentes dans un échantillon et de les classer soit dans le groupe des fièvres récurrentes (B. anserina, B. crocidurae, B. duttonii, B. hermsii, B. hispanica, B. miyamotoi, B. parkeri, B. persica, B. recurrentis, B. turicatae, B. lonestari, B. microti et B. theileri) soit dans le groupe de la maladie de Lyme (B. burgdorferi sensu stricto, B. garinii, B. afzelii, B. spielmanii, B. valaisiana, B. bissettii, B. americana, B. andersonii, B. carolinensis, B. japonica, B. lusitaniae, B. sinica, B. tanukii et B. turdi) par PCR quantitative multiplexe en temps réel. En outre, la méthode permet encore d'identifier l'espèce à laquelle appartiennent les bactéries présentes, de manière précise. Enfin, la sensibilité de la méthode est telle qu'elle permet de détecter une à dix copies dans un échantillon.

Pour cela, lors d'une première étape (a), le gène codant l'ARN ribosomique 16S des bactéries est ciblé, à l'aide d'un couple d'amorce commun à toutes les espèces. Des sondes spécifiques au groupe des borrélies responsables de la maladie de Lyme et des borrélies responsables des fièvres récurrentes, respectivement, sont utilisées afin de classer dans l'un de ces groupes les borrélies présentes dans l'échantillon.

Si cette première étape a révélé la présence de borrélies responsables de la maladie de Lyme, l'étape suivante (b) permettra de distinguer et d'identifier les borrélies burgdorferi sensu stricto, garinii et afzelii, ainsi que valaisiana et bissettii, en ciblant le gène codant la flagelline de ces espèces. Des couples d'amorces communs entre les borrélies burgdorferi sensu stricto, B. garinii et B. afzelii, et des couples d'amorces communs entre les espèces B. valaisiana et B. bissettii, sont utilisés. Des sondes spécifiques de chaque espèce sont utilisées afin d'identifier la ou les espèces détectées.

Si, par contre, la première étape a révélé la présence de borrélies appartenant au groupe des borrélies responsables des fièvres récurrentes, l'étape suivante (c) permettra d'identifier les borrélies des espèces B. hermsii et B. recurrentis en ciblant, respectivement, les gènes codant la flagelline et l'ARN ribosomique 16S de ces espèces ou d'identifier les espèces B. duttonii ou B. crocidurae en ciblant le gène codant la protéine RecA de ces espèces. Des couples d'amorces communs entre les borrélies B. hermsii et B. recurrentis, et des couples d'amorces communs entre les espèces B. duttonii ou B. crocidurae, sont utilisés. Des sondes spécifiques de chaque espèce sont utilisées afin d'identifier la ou les espèces détectées.

La méthode peut bien entendu être mise en œuvre à l'aide de toute séquences présentant au moins 90%, préférentiellement au moins 95%, plus préférentiellement au moins 98%, d'homologie avec les séquences SEQ ID NO : 1 à 4 et 8 à 27.

Avantageusement, l'étape (a) inclut l'amplification de séquences nucléotidiques codant l'actine de l'hôte à l'aide d'une paire d'amorces incluant l'amorce sens de séquence SEQ ID NO :5 et l'amorce antisens de séquence SEQ ID NO :6 et d'une sonde consistant en la séquences SEQ ID NO :7, ou de séquences présentant au moins 90% d'homologie avec les séquences SEQ ID NO : 5 à 7, dans des conditions permettant la production d'amplicons, puis la détection de la présence ou de l'absence de ladite séquence nucléotidique codant l'actine par détection de la présence d'un signal de fluorescence résultant de la formation d'amplicons.

La détection du gène de l'actine de l'hôte permet un contrôle de la qualité de l'extraction des acides nucléiques.

Selon un mode de réalisation de l'invention, les étapes (b1) et (b2) comprennent :
- une étape (b1a) consistant en l'amplification de séquences nucléotidiques cibles dudit ADN à l'aide d'une paire d'amorces incluant l'amorce sens de séquence SEQ ID NO :8 et les amorces antisens de séquences SEQ ID NO :9 et SEQ ID NO :10 et deux sondes SEQ ID NO :12 et SEQ ID NO :13,
- (b2a) la détection de la présence ou l'absence de Borrélies appartenant aux espèces B. garinii ou B. afzelii par détection de la présence d'un signal de fluorescence résultant de la formation d'amplicons, et
- une étape (b1b) consistant en l'amplification de séquences nucléotidiques cibles dudit ADN à l'aide d'une paire d'amorces incluant l'amorce sens de séquence SEQ ID NO :8 et les amorces antisens de séquences SEQ ID NO :9 et SEQ ID NO :10 et une sonde SEQ ID NO :11.
- (b2b) la détection de la présence ou l'absence de borrélies appartenant aux espèces B. burgdorferi sensu stricto par détection de la présence d'un signal de fluorescence résultant de la formation d'amplicons.

Dans ce mode de réalisation, les détections des espèces B. garinii ou B. afzelii, d'une part, et de l'espèce B. burgdorferi sensu stricto, d'autre part, sont réalisées séparément.

Avantageusement selon l'invention, l'étape (d) inclut le séquençage des amplicons générés à l'issue de chacune des étapes, c'est à dire aux étapes (a), (b) et/ou (c).

Le potentiel informatif du séquençage des amplicons obtenus peut permettre d'identifier des espèces supplémentaires par rapport à celles ciblées par les PCR multiplexes quantitatives. Notamment, peuvent être identifiées par séquençage les espèces suivantes : B. anserina, B. coriceae, B. lonestari, B. spielmanii, B. andersonii, B. japonica, B. lusitaniae, B. sinica et B. turdi.

Selon un mode de réalisation de l'invention, les conditions de l'amplification réalisée à l'étape (a) et à l'étape (c1) et (c3) sont une dénaturation initiale à 95°C durant 5 minutes puis la mise en œuvre de 45 cycles incluant chacun une dénaturation à 94°C durant 15 sec puis une hybridation et élongation à 58°C durant 45 sec.

Préférentiellement, les conditions d'amplification réalisées à l'étape (b1a) sont une dénaturation initiale à 95°C durant 5 minutes puis la mise en œuvre de 45 cycles incluant chacun une dénaturation à 94°C durant 15 sec puis une hybridation et élongation à 62°C durant 45 sec.

Préférentiellement, les conditions d'amplification réalisées à l'étape (b1b) sont une dénaturation initiale à 95°C durant 5 minutes puis la mise en œuvre de 45 cycles incluant chacun une dénaturation à 94°C durant 15 sec puis une hybridation et élongation à 60°C durant 45 sec.

Préférentiellement encore, les conditions d'amplification réalisées à l'étape (b3) sont une dénaturation initiale à 95°C durant 5 minutes puis la mise en œuvre de 45 cycles incluant chacun une dénaturation à 94°C durant 15 sec puis une hybridation et élongation à 64°C durant 45 sec.

Selon un mode de réalisation, les sondes de séquence SEQ ID NO : 3, SEQ ID NO : 4, SEQ ID NO :7, SEQ ID NO :11, SEQ ID NO :12, SEQ ID NO :13, SEQ ID NO :17, SEQ ID NO :18, SEQ ID NO :23, SEQ ID NO :24 et SEQ ID NO :27 sont marquées avec une molécule quencher sur l'extrémité 3' et un fluorophore sur l'extrémité 5', la détection d'un signal de fluorescence témoin d'une amplification étant réalisée lors de la dissociation du fluorophore et de la molécule quencher, les sondes utilisées au cours d'une même étape (a), (b1), (b3), (c1), (c3) sont marquées par des fluorochromes différents.

Avantageusement, les fluorophores sont choisis parmi 6-FAM, VIC, Cy5, HEX et l'inhibiteur de fluorescence est le BlackBerry Quencher (BBQ) ou une molécule MGB (Minor Groove Binder) associée à un quencher non fluorescent (NFQ, Eclipse).

Selon une caractéristique de l'invention, l'échantillon est du sang, de l'urine, de la salive, un tissu cellulaire.

Décrit est encore une séquence nucléotidique comprenant une séquence nucléotidique choisie parmi : SEQ ID NO :1, SEQ ID NO :2, SEQ ID NO :5 SEQ ID NO :6, SEQ ID NO :8, SEQ ID NO :9, SEQ ID NO :10, SEQ ID NO :14, SEQ ID NO :15, SEQ ID NO :16, SEQ ID NO :19, SEQ ID NO :20, SEQ ID NO :21, SEQ ID NO :22, SEQ ID NO :25, SEQ ID NO :26, SEQ ID NO :28, SEQ ID NO :29, SEQ ID NO :30, SEQ ID NO :31, SEQ ID NO :32, SEQ ID NO :33, SEQ ID NO :34, SEQ ID NO :35, SEQ ID NO :36, SEQ ID NO : 37, ou toute séquence présentant une homologie d'au moins 90%, préférentiellement d'au moins 95%, plus préférentiellement d'au moins 98%, avec l'une desdites séquences, ainsi que leur séquence complémentaire respective.

L'invention concerne encore une sonde constituée d'une séquence nucléotidique choisie parmi : SEQ ID NO : 3, SEQ ID NO : 4, SEQ ID NO :7, SEQ ID NO :11, SEQ ID NO :12, SEQ ID NO :13, SEQ ID NO :17, SEQ ID NO :18, SEQ ID NO :23, SEQ ID NO :24 et SEQ ID NO:27, de même est décrite toute séquence présentant une homologie d'au moins 90%, préférentiellement d'au moins 95%, plus préférentiellement d'au moins 98%, avec l'une desdites séquences, ainsi que leur séquence complémentaire respective.

Selon un mode de réalisation, les extrémités 5' des sondes sont marquées par le 6-FAM, le VIC, le CY5 ou le HEX.

L'invention concerne encore un kit pour la détection de la présence de borrélies dans un échantillon humain ou animal et la discrimination et l'identification des espèces de borrélies, par PCR multiplexes quantitatives, comprenant :
(i) une paire d'amorces incluant l'amorce sens de séquence SEQ ID NO :1 et l'amorce antisens de séquence SEQ ID NO :2 et deux sondes consistant en les séquences SEQ ID NO :3 et SEQ ID NO :4, ou toute séquence présentant une homologie d'au moins 90%, préférentiellement d'au moins 95%, plus préférentiellement d'au moins 98%, avec l'une desdites séquences, ou toute séquence complémentaire,
(ii) des paires d'amorces formées par l'amorce sens de séquence SEQ ID NO :8 et les amorces antisens de séquence SEQ ID NO :9 et SEQ ID NO :10, et trois sondes consistant en les séquences SEQ ID NO :11, SEQ ID NO :12 et SEQ ID NO :13, ou toute séquence présentant une homologie d'au moins 90%, préférentiellement d'au moins 95%, plus préférentiellement d'au moins 98%, avec l'une desdites séquences, ou toute séquence complémentaire,
(iii) des paires d'amorces formées par l'amorce sens de séquence SEQ ID NO :14, l'amorce sens de séquence SEQ ID NO :15 et l'amorce antisens de séquence SEQ ID NO :16, et deux sondes consistant en les séquences SEQ ID NO :17 et SEQ ID NO :18, ou toute séquence présentant une homologie d'au moins 90%, préférentiellement d'au moins 95%, plus préférentiellement d'au moins 98%, avec l'une desdites séquences, ou toute séquence complémentaire,
(iv) une paire d'amorces incluant l'amorce sens de séquence SEQ ID NO :19 et l'amorce antisens de séquence SEQ ID NO :20 et une paire d'amorces incluant l'amorce sens de séquence SEQ ID NO :21 et l'amorce antisens de séquence SEQ ID NO :22, et deux sondes consistant en les séquences SEQ ID NO :23 et SEQ ID NO :24, ou toute séquence présentant une homologie d'au moins 90%, préférentiellement d'au moins 95%, plus préférentiellement d'au moins 98%, avec l'une desdites séquences, ou toute séquence complémentaire,
(v) une paire d'amorces incluant l'amorce sens de séquence SEQ ID NO :25 et l'amorce antisens de séquence SEQ ID NO :26 et une sonde consistant en la séquence SEQ ID NO :27, ou toute séquence présentant une homologie d'au moins 90%, préférentiellement d'au moins 95%, plus préférentiellement d'au moins 98%, avec l'une desdites séquences, ou toute séquence complémentaire,
(vi) ainsi que tous réactifs nécessaires à la réalisation de PCR quantitative multiplexe,
les sondes étant marquées avec une molécule quencher sur l'extrémité 3' et un fluorophore sur l'extrémité 5'.

L'invention concerne enfin, l'utilisation d'un kit tel que défini précédemment pour la détection de la présence de borrélies dans un échantillon humain ou animal et de discrimination des espèces de borrélies par PCR multiplexes quantitatives.

Le kit selon l'invention est utilisé pour la détection des espèces de borrélies B. burgdorferi sensu stricto, B. garinii, B. afzelii, B. valaisiana et B. bissettii du groupe provoquant la maladie de Lyme, et des espèces B. hermsii, B. recurrentis, B. duttonii ou B. crocidurae du groupe provoquant les fièvres récurrentes. En outre, il permet également l'identification des espèces B. anserina, B. coriceae, B. lonestari, B. spielmanii, B. andersonii, B. japonica, B. lusitaniae, B. sinica et B. turdi, par la technique de séquençage.

A titre d'exemples non limitatifs, l'échantillon peut provenir de l'homme ou d'animaux tels que des animaux domestiques, d'élevage ou encore sauvages.

L'invention est illustrée dans ce qui suit, à l'aide d'un exemple de réalisation de l'invention qui se veut illustratif mais non limitatif.

### EXEMPLE

### Etape (a)

L'étape (a) permet de détecter la présence de borrélies dans un échantillon et de déterminer si elles appartiennent au groupe des fièvres récurrentes (B. anserina, B. crocidurae, B. duttonii, B. hermsii, B. hispanica, B. miyamotoi, B. parkeri, B. persica, B. recurrentis, B. turicatae, B. lonestari, B. microti et B. theileri) ou au groupe de la maladie de Lyme (B. burgdorferi sensu stricto, B. garinii, B. afzelii, B. spielmanii, B. valaisiana, B. bissettii, B. americana, B. andersonii, B. carolinensis, B. japonica, B. lusitaniae, B. sinica, B. tanukii et B. turdi). L'étape (a) est réalisée par PCR quantitative multiplexe en temps réel. Pour cela le gène codant l'ARN ribosomique 16S de ces bactéries est ciblé. Le gène de l'actine de l'hôte est utilisé comme standard interne.

Un travail de recherche a été effectué afin de déterminer des amorces permettant une détection spécifique des bactéries borrélies et qui soit commune à toutes les espèces citées ci-dessus. Des sondes permettant la distinction entre le groupe des fièvres récurrentes (« sonde Fièvres ») et le groupe de la maladie de Lyme (« sonde Lyme ») ont été déterminées. Pour cela, 84 séquences du gène codant l'ARN 16S de 25 espèces de borrélies ont été alignées. Dix amorces sens et 7 amorces anti-sens ont été déterminées, et le meilleur couple en termes de spécificité, d'absence de formation de dimères d'amorces, de taille d'amplicon généré a été choisi puis validé en Q-PCR. Deux sondes spécifiques des deux sous-groupes de borrélies ont été définies et testées.

Par ailleurs, le séquençage de l'amplicon du gène codant l'ARN 16S produit à cette étape (a) peut permettre d'identifier trois espèces de borrélies non recherchées dans les étapes ultérieures. Ces espèces sont B. anserina, B. coriceae et B. lonestari.

### Séquences des amorces et sondes spécifiques des borrélies à l'étape (a):

Les séquences du couple d'amorces et des sondes spécifiques des deux groupes de borrélies sont indiquées dans le Tableau 1.

**Tableau 1**

| Gène cible | N° | Nom de la séquence | séquence |
|---|---|---|---|
| ARN 16S | SEQ ID NO :1 | Borrelia A sens | 5'- CGT TGT TCG GGA TTA TTG G -3' |
| | SEQ ID NO:2 | Borrelia A anti-sens | 5'- CAG ATT CCA CCC TTA CAC CAG -3' |
| | SEQ ID NO :3 | Sonde Lyme | 5'- 6-FAM - ATA GAG GAA GTT A - NFQ/MGB -3' |
| | SEQ ID NO :4 | Sonde Fièvres | 5'- VIC - CAT GAC TAG AGT CT - NFQ/MGB-3' |
| actine | SEQ ID NO :5 | Actine sens: | 5'- ACC CAC ACT GTG CCC ATC TA -3' |
| | SEQ ID NO :6 | Actine anti-sens | 5'- CTT GAT GTC ACG CAC GAT TT -3' |
| | SEQ ID NO :7 | Sonde actine | 5'-Cy5-GGC TAC AGC TTC ACC ACC AC -BBQ -3' |

### Matériel et méthode :

### 1-Préparation des échantillons :

L'ADN servant aux Q-PCR est extrait à partir du prélèvement (sang, liquide céphalorachidien, urines, liquide articulaire, biopsies de tissu...) avec le kit QIAamp DNA Mini Kit (Réf. 51304, Qiagen, France) selon le protocole indiqué dans le manuel d'utilisation. Les ADN sont élués dans l'eau et dosés par spectrométrie avant utilisation. La qualité de l'extraction est vérifiée par le rapport D.O.260/D.O.280.

### 2-Conditions de Q-PCR :

Deux contrôles positifs ont été réalisés, ils correspondent aux séquences nucléiques des borrélies amplifiées par les amorces définies ici et contenues dans un vecteur plasmidique PexA. Ils ont été produits par une synthèse de gène par envoi des séquences à la société Eurofins. Une gamme de calibration à 7 points allant de 0,25 ng (10⁸ copies) à 0,25 ag (0.1 copie) de ces contrôles positifs permet d'obtenir une quantification en nombre de copies des borrélies présentes dans un échantillon.

### 3- Le mix nécessaire à la réaction de Q-PCR de l'étape (a) est préparé comme indiqué dans le Tableau 2:

**Tableau 2**

| | Quantité | Concentration finale |
|---|---|---|
| Borrelia A sens, 20 µM | 0,25 µl | 200 nM |
| Borrelia A anti-sens, 20 µM | 0,25 µl | 200 nM |
| Sonde Lyme, 20 µM | 0,25 µl | 200 nM |
| Sonde Fièvres, 20 µM | 0,25 µl | 200 nM |
| Mix iTAQ supermix (Réf. 1725131, Biorad) | 12,5 µl | 1X |
| Eau (Réf. BE51200, Lonza) | qsp 20 µl | - |

Par réaction (point de gamme, échantillons ou contrôle négatif), 20 µl de mix ainsi que 5 µl d'échantillon (50 ng à 200 ng), d'eau (pour le contrôle négatif) ou de point de gamme sont déposés (volume réactionnel final de 25 µl).

Les conditions d'amplification sont les suivantes : dénaturation initiale à 95°C durant 5 minutes puis la mise en œuvre de 45 cycles incluant chacun une dénaturation à 94°C durant 15 sec puis une hybridation et élongation à 58°C durant 45 sec. La détection des fluorochromes FAM et VIC est sélectionnée.

Le mix nécessaire à la réaction de Q-PCR pour la détection de l'actine est préparé comme suit:

**Tableau 3**

| | Quantité | Concentration finale |
|---|---|---|
| Actine sens, 20 µM | 0,125 µl | 100 nM |
| Actine anti-sens, 20 µM | 0,125 µl | 100 nM |
| Sonde Actine, 20 µM | 0,125 µl | 100 nM |
| Mix iTAQ supermix (Réf. 1725131, Biorad) | 12,5 µl | 1X |
| Eau (Réf. BE51200, Lonza) | qsp 20 µl | - |

Par réaction (point de gamme, échantillons ou contrôle négatif), 20 µl de mix ainsi que 5 µl d'échantillon (50 ng à 200 ng), d'eau (pour le contrôle négatif) ou de point de gamme sont déposés (volume réactionnel final de 25 µl).

Les conditions d'amplification sont 95°C, 5 minutes puis 40 cycles chacun de dénaturation à 94°C durant 15 sec puis hybridation et élongation à 58°C, 30 sec.

La détection du fluorochrome Cy5 est sélectionnée.

### 4-Résultats

Dans ces conditions, la limite de détection des borrélies du groupe de la maladie de Lyme et des borrélies du groupe des Fièvres récurrentes est de 1 copie (soit 2.5 ag).

Les amplicons des borrélies du groupe de la maladie de Lyme et des borrélies du groupe des fièvres récurrentes obtenus sont de 153 paires de bases. Les séquences de ces produits de Q-PCR sont les suivantes (Tableau 4):

**Tableau 4**

| N° | Nom de la séquence | séquence |
|---|---|---|
| SEQ ID NO :28 | amplicon des borrélies du groupe maladie de Lyme | |
| SEQ ID NO :29 | amplicon des borrélies du groupe des Fièvres récurrentes | |

### Etape (b)

Si l'étape (a) a permis de déterminer la présence de borrélies du groupe de la maladie de Lyme alors l'étape (b) est réalisée pour déterminer, respectivement, la présence des espèces B. burgdorferi sensu stricto, B. garinii ou B. afzelii (étapes (b1), (b1a), (b1b) ou (b2)), et des espèces B. valaisiana ou B. bissettii (étapes (b3) et (b4)), appartenant à ce groupe, par PCR quantitative multiplexe en temps réel. Pour cela le gène codant la flagelline de ces bactéries est ciblé.

Afin de mettre en place les étapes (b1) ou (b1a) et (b1b), un travail de recherche a été effectué pour déterminer un couple d'amorces permettant la détection des espèces B. burgdorferi sensu stricto, B. garinii et B. afzelii ainsi que 3 sondes permettant la reconnaissance spécifique de chacune de ces espèces. Ces 3 sondes sont nommées « sonde Burdg ss », « sonde Garinii » et « sonde Afzelii ». Ce travail s'est initialement basé sur les différences de séquences de ces 3 espèces dans le gène codant la flagelline, qui sont décrites l'article de Picken R. (1992, Journal of Clinical Microbiology, 30:99-114).

Pour cela, 57 séquences du gène codant la flagelline de 11 espèces de borrélies ont été alignées. Les meilleures séquences d'amorces et de sondes en termes de spécificité, d'absence de formation de dimères d'amorces, de taille d'amplicon généré ont été choisies puis validées en Q-PCR. Les sondes ainsi déterminées ici ne ciblent pas les mêmes différences de séquences que celles de Picken R.. Les sondes décrites par Picken R. étant en revanche très longues (de 49 à 52 paires de bases), les sondes Burdg ss et Afzelii décrites ici ont, respectivement, 5 et 8 nucléotides en commun avec celles de Picken R. (c'est-à-dire que les extrémités 3' des sondes de Picken R. ont 5 à 8 bases communes avec les extrémités 5' de deux de nos sondes). Les deux sondes permettant la reconnaissance de l'espèce garinii n'ont aucune base commune.

Le séquençage des amplicons du gène de la flagelline produit à l'étape (b1) ou (b1a) et (b1b) peut permettre de reconnaître 6 espèces de borrélies non recherchées dans les étapes suivantes. Ces espèces sont B. spielmanii, B. andersonii, B. japonica, B. lusitaniae, B. sinica et B. turdi.

En parallèle, un travail de recherche a été effectué pour déterminer un couple d'amorces permettant la détection des espèces B. valaisiana ou B. bissettii ainsi que 2 sondes (« sonde Valaisiana » et « sonde Bissettii ») permettant la reconnaissance spécifique de chacune de ces espèces pour la mise en œuvre de l'étape (b3) et (b4). Ce travail s'est inspiré des différences de séquences de ces 2 espèces dans le gène codant la flagelline qui sont décrites dans l'article de Jauhlac B. (2000, Journal of Clinical Microbiology, 38:1895-1900). Pour cela, 57 séquences du gène codant la flagelline de 11 espèces de borrélies ont été alignées. Les meilleures séquences d'amorces et de sondes en termes de spécificité, d'absence de formation de dimères d'amorces, de taille d'amplicon généré ont été choisies puis validées en Q-PCR. La sonde déterminée ici et permettant la reconnaissance de l'espèce valaisiana a 15 bases en commun avec celles de Jauhlac B. qui en fait 27. Les deux sondes permettant la reconnaissance de l'espèce bissetti n'ont aucune base commune.

### Séquences des amorces et sondes spécifiques des espèces de borrélies du groupe de la maladie de Lyme pour les étapes (b1) ou (b1a) et (b1b) :

Les séquences du couple d'amorces et des sondes spécifiques des espèces B. burgdorferi sensu stricto, B. garinii ou B. afzelii sont indiquées dans la Tableau 5:

**Tableau 5**

| Gène cible | N° | Nom de la séquence | séquence |
|---|---|---|---|
| flagelline | SEQ ID NO :8 | Borrelia B sens | 5'- GGA GCA AAT CAA GAT GAR GC -3' |
| | SEQ ID NO :9 | Borrelia B anti-sens n1 | 5'- ACA GGA GAA TTA ACT CCA CCY TG-3' |
| | SEQ ID NO :10 | Borrelia B anti-sens n2 | 5'- AGG AGA ATT AAC TCC GCC TTG -3' |
| | SEQ ID NO :11 | Sonde Burgd ss | |
| flagelline | SEQ ID NO :12 | Sonde Afzelii | 5'- VIC -AGG GTG CTC AAG AAG A - NFQ/MGB -3' |
| | SEQ ID NO :13 | Sonde Garinii | |

### Les séquences du couple d'amorces et des sondes spécifiques des espèces B. valaisiana ou B. bissettii pour l'étape (b3) sont indiquées dans le Tableau 6:

**Tableau 6**

| Gène cible | N° | Nom de la séquence | séquence |
|---|---|---|---|
| flagelline | SEQ ID NO :14 | Borrelia C sens n1 | 5'- ACC AAG ATG AAG CTA TTG CTG TAA -3' |
| | SEQ ID NO :15 | Borrelia C sens n2 | 5'- AAG ATG AGG CGA TTG CTG TAA -3' |
| | SEQ ID NO :16 | Borrelia C anti-sens | 5'- CAG GTG CTG GYT GTT GAG C -3' |
| | SEQ ID NO :17 | Sonde Valaisiana | 5'-VIC - CTA CAC CTG TTC AAG AA - NFQ/MGB -3' |
| flagelline | SEQ ID NO :18 | Sonde Bissettii | 5'- 6-FAM - TCG CAA ATC TTT TCT CT - NFQ/MGB -3' |

### Protocole:

### 1-Préparation des échantillons

La préparation des échantillons est identique à celle réalisée à l'étape (a).

### 2-Conditions de Q-PCR

Cinq contrôles positifs ont été réalisés. Ils correspondent aux séquences nucléiques des borrélies amplifiées par les deux couples d'amorces définies ici et contenues dans un vecteur plasmidique PexA. Une gamme de calibration à 7 points allant de 0,25 ng (10⁸ copies) à 0,25 ag (0.1 copie) de ces contrôles positifs permet d'obtenir une quantification en nombre de copies des borrélies présentes dans un échantillon.

### 3-Etapes (b1a) et (b1b) :

Les étapes (b1a) et (b1b) ont pour but la détection de la présence ou l'absence de borrélies appartenant aux espèces B. burgdorferi sensu stricto, B. garinii ou B. afzelii par détection de la présence ou absence d'un signal de fluorescence résultant de la formation d'amplicons.

L'étape (b1a) a pour but l'amplification de séquences nucléotidiques cibles à l'aide d'une paire d'amorces incluant l'amorce sens de séquence SEQ ID NO :8 et les amorces antisens de séquence SEQ ID NO :9 et SEQ ID NO :10 et de deux sondes consistant en les séquences SEQ ID NO :12 et SEQ ID NO :13.

Le mix nécessaire à la réaction de Q-PCR de l'étape (b1a) est préparé comme suit:

**Tableau 7**

| | Quantité | Concentration finale |
|---|---|---|
| Borrelia B sens, 20 µM | 0,25 µl | 200 nM |
| Borrelia B anti-sens n1, 20 µM | 0,25 µl | 200 nM |
| Borrelia B anti-sens n2, 20 µM | 0,25 µl | 200 nM |
| Sonde Afzelii, 20 µM | 0,25 µl | 200 nM |
| Sonde Garinii, 20 µM | 0,25 µl | 200 nM |
| Mix iTAQ supermix (Réf. 1725131, Biorad) | 12,5 µl | 1X |
| Eau (Réf. BE51200, Lonza) | qsp 20 µl | - |

Par réaction (point de gamme, échantillons ou contrôle négatif), 20 µl de mix ainsi que 5 µl d'échantillon, d'eau (pour le contrôle négatif) ou de point de gamme sont déposés (volume réactionnel final de 25 µl).

Les conditions d'amplification sont les suivantes : dénaturation initiale à 95°C durant 5 minutes puis la mise en œuvre de 45 cycles incluant chacun une dénaturation à 94°C durant 15 sec puis une hybridation et élongation à 62°C durant 45 sec.

La détection des fluorochromes FAM, et VIC est sélectionnée pour mettre en œuvre l'étape (b1a).

L'étape (b1b) a pour but l'amplification de séquences nucléotidiques cibles à l'aide d'une paire d'amorces incluant l'amorce sens de séquence SEQ ID NO :8 et les amorces antisens de séquence SEQ ID NO :9 et SEQ ID NO :10 et d'une sonde consistant en la séquence SEQ ID NO :11.

Le mix nécessaire à la réaction de Q-PCR de l'étape (b1b) est préparé comme suit:

**Tableau 8**

| | Quantité | Concentration finale |
|---|---|---|
| Borrelia B sens, 20 µM | 0,25 µl | 200 nM |
| Borrelia B anti-sens n1, 20 µM | 0,25 µl | 200 nM |
| Borrelia B anti-sens n2, 20 µM | 0,25 µl | 200 nM |
| Sonde Burdorferi ss, 20 µM | 0,25 µl | 50 nM |
| Mix iTAQ supermix (Réf. 1725131, Biorad) | 12,5 µl | 1X |
| Eau (Réf. BE51200, Lonza) | qsp 20 µl | - |

Par réaction (point de gamme, échantillons ou contrôle négatif), 20 µl de mix ainsi que 5 µl d'échantillon, d'eau (pour le contrôle négatif) ou de point de gamme sont déposés (volume réactionnel final de 25 µl).

Les conditions d'amplification sont les suivantes : dénaturation initiale à 95°C durant 5 minutes puis la mise en œuvre de 45 cycles incluant chacun une dénaturation à 94°C durant 15 sec puis une hybridation et élongation à 60°C durant 45 sec.

La détection du fluorochrome Cy5 est sélectionnée pour mettre en œuvre l'étape (b1b).

Dans une variante de l'invention, les étapes b1a et b1b sont réalisées concomitamment en une seule étape b1.

### 4- étapes (b3) et (b4)

L'étape (b3) a pour but l'amplification de séquences nucléotidiques cibles dudit ADN à l'aide d'une paire d'amorces incluant l'amorce sens de séquence SEQ ID NO :14, l'amorce sens de séquence SEQ ID NO :15 et l'amorce antisens de séquence SEQ ID NO :16 et de deux sondes consistant en les séquences SEQ ID NO :17 et SEQ ID NO :18, dans des conditions permettant la production d'amplicons.

L'étape (b3) a pour but la détection de la présence ou l'absence de Borrélies appartenant aux espèces B. valaisiana ou B. bissettii par détection de la présence ou absence d'un signal de fluorescence résultant de la formation d'amplicons.

Le mix nécessaire à la réaction de Q-PCR de l'étape (b3) est préparé comme suit:

**Tableau 9**

| | Quantité | Concentration finale |
|---|---|---|
| Borrelia C sens n1, 20 µM | 0,25 µl | 200 nM |
| Borrelia C sens n2, 20 µM | 0,25 µl | 200 nM |
| Borrelia C anti-sens, 20 µM | 0,25 µl | 200 nM |
| Sonde Valaisiana, 20 µM | 0,25 µl | 200 nM |
| Sonde Bissettii, 20 µM | 0,25 µl | 200 nM |
| Mix iTAQ supermix (Réf. 1725131, Biorad) | 12,5 µl | 1X |
| Eau (Réf. BE51200, Lonza) | qsp 20 µl | - |

Par réaction (point de gamme, échantillons ou contrôle négatif), 20 µl de mix ainsi que 5 µl d'échantillon, d'eau (pour le contrôle négatif) ou de point de gamme sont déposés (volume réactionnel final de 25 µl).

Les conditions d'amplification sont les suivantes : dénaturation initiale à 95°C durant 5 minutes puis la mise en œuvre de 45 cycles incluant chacun une dénaturation à 94°C durant 15 sec puis une hybridation et élongation à 64°C durant 45 sec.

La détection des fluorochromes FAM et VIC est sélectionnée à l'étape (b4).

### 5-Résultats

Dans ces conditions, la limite de détection des espèces B. burgdorferi sensu stricto, B. garinii et B. afzelii est de 1 copie (soit 2.5 ag) et celle des espèces B. valaisiana et B. bissettii est de 1 copie (soit 2.5 ag).

Les amplicons des espèces de borrélies burgdorferi sensu stricto, afzelii et garinii obtenus sont de 180 ou 182 paires de bases. Les amplicons des espèces de borrélies valaisiana et bissettii obtenus sont de 135 et 138 paires de bases, respectivement. Les séquences de ces produits de Q-PCR sont les suivantes (Tableau 10):

**Tableau 10**

| N° | Nom de la séquence | séquence |
|---|---|---|
| SEQ ID NO :30 | amplicon des borrélies burgdorferi sensu stricto | |
| SEQ ID NO :31 | amplicon des borrélies afzelii | |
| SEQ ID NO :32 | amplicon des borrélies garinii | |
| SEQ ID NO :33 | amplicon des borrélies valaisiana | |
| SEQ ID NO :34 | amplicon des borrélies bissettii | |

### Etape (c)

Si l'étape (a) a permis de déterminer la présence de borrélies du groupe des fièvres récurrentes, alors l'étape (c) est réalisée pour déterminer, respectivement, la présence des espèces B. hermsii et B. recurrentis (étapes (c1) et (c2)), et des espèces B. duttonii ou B. crocidurae (étape (c3) et (c4)), par PCR quantitative multiplexe en temps réel.

B. hermsii est distingué en ciblant le gène codant la flagelline et B. recurrentis en ciblant celui codant l'ARNr 16S. B. duttonii ou B. crocidurae sont identifiés en ciblant le gène RecA (protéine de réparation de l'ADN).

Afin de mettre en place l'étape (c1), un travail de recherche a été effectué pour déterminer un couple d'amorces (Borrelia D fla sens et anti-sens) et une sonde spécifique (« sonde Hermsii ») permettant la détection de l'espèce B. hermsii. Pour cela 59 séquences du gène codant la flagelline de 12 espèces de borrélies ont été alignées et comparées. Egalement, un couple d'amorces (Borrelia D 16S sens et anti sens) et une sonde spécifique permettant la détection de l'espèce B. recurrentis (« sonde Recurrentis ») ont été déterminés. Pour cela 61 séquences du gène codant l'ARNr 16S de 23 espèces de borrélies ont été alignées. Les meilleures séquences d'amorces et de sondes en termes de spécificité, d'absence de formation de dimères d'amorces, de taille d'amplicons générés ont été choisies puis validées en Q-PCR.

Sur la base de polymorphismes décrits dans le gène RecA de B. duttonii par Cutler S., un travail de recherche a également été effectué sur le gène RecA afin de déterminer un couple d'amorces et une sonde spécifique (« sonde Dut/Croc ») pour la détection des espèces B. duttonii ou B. crocidurae et la mise en œuvre de l'étape (c3). Ce travail est basé sur l'alignement de 62 séquences du gène RecA provenant de 10 espèces de borrélies. Les meilleures séquences d'amorces et de sondes en termes de spécificité, d'absence de formation de dimères d'amorces, de taille d'amplicon généré ont été choisies puis validées en Q-PCR.

### Séquences des amorces et sondes spécifiques des espèces de borrélies du groupe des fièvres récurrentes:

Les séquences des couples d'amorces et des sondes spécifiques des espèces B. hermsii ou B. recurrentis pour l'étape (c1) sont indiquées dans la Tableau 11 :

**Tableau 11**

| Gène cible | N° | Nom de la séquence | séquence |
|---|---|---|---|
| flagelline | SEQ ID NO :19 | Borrelia D fla sens: | 5'-AAA TCT TTT TGC AGG TGA AGG -3' |
| | SEQ ID NO :20 | Borrelia D fla anti-sens | 5'- CAT CAA CAG CGG TTG TAA CAT -3' |
| ARNr16S | SEQ ID NO :21 | Borrelia D 16S sens | 5'- GGA AAT GAC AAG GTG ATG ACG -3' |
| | SEQ ID NO :22 | Borrelia D 16S anti-sens | 5'- GCA GTT TCT AGC ATA GCT CCA CA-3' |
| flagelline | SEQ ID NO :23 | Sonde Hermsii | 5'- 6-FAM - AAG AGA TAG GAC AGC A - NFQ/EQ -3' |
| ARNr16S | SEQ ID NO :24 | Sonde Recurrentis | 5'-HEX - AGT CCC GGC TAA TT - NFQ/EQ -3' |

Les séquences du couple d'amorces et de la sonde spécifique des espèces B. duttonii et B. crocidurae pour l'étape (c3) sont indiquées dans la Tableau 12 :

**Tableau 12**

| Gène cible | N° | Nom de la séquence | séquence |
|---|---|---|---|
| RecA | SEQ ID NO :25 | Borrelia E sens: | 5'- GAT GGC GAG ATG GGT GA T ACT -3' |
| | SEQ ID NO :26 | Borrelia E anti-sens | 5'- GCA TTT CCT CCA GTA GTA GTC TCT G -3' |
| | SEQ ID NO :27 | Sonde Dut/Croc | 5'- HEX - AGC CAT ACT TTC A - NFQ/EQ -3' |

### Protocole:

### 1-Préparation des échantillons

La préparation des échantillons est identique à celle réalisée à l'étape (a).

### 2-Conditions de Q-PCR

Trois contrôles positifs ont été réalisés. Ils correspondent aux séquences nucléiques des borrélies amplifiées par les trois couples d'amorces définies ici et contenues dans un vecteur plasmidique PexA. Ils ont été produits par une synthèse de gène par envoi des séquences à la société Eurofins. Une gamme de calibration à 7 points allant de 0,25 ng (10⁸ copies) à 0,25 ag (0.1 copie) de ces contrôles positifs permet d'obtenir une quantification en nombre de copies des borrélies présentes dans un échantillon.

### 3-Etapes (c1) et (c2) :

L'étape (c1) a pour but l'amplification de séquences nucléotidiques cibles dudit ADN à l'aide d'une paire d'amorces incluant l'amorce sens de séquence SEQ ID NO:19 et l'amorce antisens de séquence SEQ ID NO :20 et d'une paire d'amorces incluant l'amorce sens de séquence SEQ ID NO :21 et l'amorce antisens de séquence SEQ ID NO :22 et de deux sondes consistant en les séquences SEQ ID NO :23 et SEQ ID NO :24.

L'étape (c2) a pour but la détection de la présence ou l'absence de borrélies appartenant aux espèces espèces B. hermsii et B. recurrentis, par détection de la présence ou absence d'un signal de fluorescence résultant de la formation d'amplicons.

Le mix nécessaire à la réaction de Q-PCR de l'étape (c1) est préparé comme suit:

**Tableau 13**

| | Quantité | Concentration finale |
|---|---|---|
| Borrelia D fla sens, 20 µM | 0,25 µl | 200 nM |
| Borrelia D fla anti-sens, 20 µM | 0,25 µl | 200 nM |
| Borrelia D 16S sens, 20 µM | 0,25 µl | 200 nM |
| Borrelia D 16S anti-sens, 20 µM | 0,25 µl | 200 nM |
| Sonde Hermsii, 20 µM | 0,25 µl | 200 nM |
| Sonde Recurrentis, 20 µM | 0,25 µl | 200 nM |
| Mix iTAQ supermix (Réf. 1725131, Biorad) | 12,5 µl | 1X |
| Eau (Réf. BE51200, Lonza) | qsp 20 µl | - |

Par réaction (point de gamme, échantillons ou contrôle négatif), 20 µl de mix ainsi que 5 µl d'échantillon, d'eau (pour le contrôle négatif) ou de point de gamme sont déposés (volume réactionnel final de 25 µl).

Les conditions d'amplification sont une dénaturation initiale à 95°C durant 5 minutes puis la mise en œuvre de 45 cycles incluant chacun une dénaturation à 94°C durant 15 sec puis une hybridation et élongation à 58°C durant 45 sec.

La détection des fluorochromes FAM et HEX est sélectionnée à l'étape (c2).

### 4- Etapes (c3) et (c4) :

L'étape (c3) a pour but l'amplification de séquences nucléotidiques cibles à l'aide d'une paire d'amorces incluant l'amorce sens de séquence SEQ ID NO :25 et l'amorce antisens de séquence SEQ ID NO :26 et d'une sonde consistant en la séquence SEQ ID NO :27, dans des conditions permettant la production d'amplicons.

L'étape (c4) a pour but la détection de la présence ou l'absence de borrélies appartenant aux espèces B. crocidurae par détection de la présence ou absence d'un signal de fluorescence résultant de la formation d'amplicons.

Le mix nécessaire à la réaction de Q-PCR de l'étape (c3) est préparé comme suit:

**Tableau 14**

| | Quantité | Concentration finale |
|---|---|---|
| Borrelia E sens, 20 µM | 0,25 µl | 200 nM |
| Borrelia E anti-sens, 20 µM | 0,25 µl | 200 nM |
| Sonde Dut/Croc, 20 µM | 0,25 µl | 200 nM |
| Mix iTAQ supermix (Réf. 1725131, Biorad) | 12,5 µl | 1X |
| Eau (Réf. BE51200, Lonza) | qsp 20 µl | - |

Par réaction (point de gamme, échantillons ou contrôle négatif), 20 µl de mix ainsi que 5 µl d'échantillon, d'eau (pour le contrôle négatif) ou de point de gamme sont déposés (volume réactionnel final de 25 µl).

Les conditions d'amplification sont 95°C, 5 minutes puis 45 cycles incluant chacun une dénaturation à 94°C durant 15 sec, puis une hybridation et élongation à 58°C durant 45 sec.

A l'étape (c4), la détection des fluorochromes VIC ou HEX est sélectionnée.

### 5-Résultats

Dans ces conditions, la limite de détection des espèces B. hermsii et B. recurrentis est de 10 copies (soit 25 ag) et celle des espèces B. duttonii et B. crocidurae est de 10 copies (soit 25 ag).

Les amplicons des espèces de borrélies hermsii et recurrentis obtenus sont de 146 et 184 paires de bases, respectivement. Les amplicons des espèces de borrélies duttonii et crocidurae obtenus sont de 176 paires de bases.

Les séquences de ces produits de Q-PCR sont les suivantes:

**Tableau 15**

| N° | Nom de la séquence | séquence |
|---|---|---|
| SEQ ID NO: 35 | amplicon des borrélies hermsii | |
| SEQ ID NO: 36 | amplicon des borrélies recurrentis | |
| SEQ ID NO : 37 | amplicon des borrélies duttonii et crocidurae | |

## Revendications

1. Méthode de détection de la présence de borrélies dans un échantillon humain ou animal et d'identification d'espèces de borrélies, par PCR multiplexes quantitatives, comprenant :
(a) une étape de détection de la présence de borrélies dans l'échantillon et de distinction desdites borrélies entre le groupe provoquant la maladie de Lyme et le groupe provoquant des fièvres récurrentes, ladite étape consistant en :
- l'extraction d'ADN dudit échantillon,
- l'amplification de séquences nucléotidiques cibles dudit ADN à l'aide d'une paire d'amorces incluant l'amorce sens de séquence SEQ ID NO :1 et l'amorce antisens de séquence SEQ ID NO :2 et de deux sondes consistant en les séquences SEQ ID NO :3 et SEQ ID NO :4, dans des conditions permettant la production d'amplicons, et,
- la détection de la présence ou l'absence de borrélies du groupe provoquant la maladie de Lyme ou du groupe provoquant des fièvres récurrentes par détection de la présence d'un signal de fluorescence résultant de la formation d'amplicons, puis,
si un signal est détecté à l'étape (a) indiquant la présence de borrélies du groupe de la maladie de Lyme,
(b) une étape de détection et de distinction, dans l'échantillon, de la présence des espèces B. burgdorferi sensu stricto, B. garinii ou B. afzelii et des espèces B. valaisiana ou B. bissettii, ladite étape consistant en :
- à partir de l'ADN extrait dudit échantillon,
- (b1) l'amplification de séquences nucléotidiques cibles dudit ADN à l'aide de paires d'amorces formées par l'amorce sens de séquence SEQ ID NO :8 et les amorces antisens de séquences SEQ ID NO :9 et SEQ ID NO :10 et de trois sondes consistant en les séquences SEQ ID NO :11, SEQ ID NO :12 et SEQ ID NO :13, dans des conditions permettant la production d'amplicons, et,
- (b2) la détection de la présence ou l'absence de borrélies appartenant aux espèces B. burgdorferi sensu stricto, B. garinii ou B. afzelii par détection de la présence d'un signal de fluorescence résultant de la formation d'amplicons, et
- (b3) l'amplification de séquences nucléotidiques cibles dudit ADN à l'aide de paires d'amorces formées par l'amorce sens de séquence SEQ ID NO :14, l'amorce sens de séquence SEQ ID NO :15 et l'amorce antisens de séquence SEQ ID NO :16, et de deux sondes consistant en les séquences SEQ ID NO :17 et SEQ ID NO :18, dans des conditions permettant la production d'amplicons, et ,
- (b4) la détection de la présence ou l'absence de borrélies appartenant aux des espèces B. valaisiana ou B. bissettii par détection de la présence d'un signal de fluorescence résultant de la formation d'amplicons,
ou, si un signal est détecté à l'étape (a) indiquant la présence de Borrélies du groupe des fièvres récurrentes,
(c) une étape de détection et de distinction, dans l'échantillon, de la présence des espèces B. hermsii et B. recurrentis, et des espèces B. duttonii ou B. crocidurae du groupe provoquant les fièvres récurrentes, ladite étape consistant en :
- à partir de l'ADN extrait dudit échantillon
- (c1) l'amplification de séquences nucléotidiques cibles dudit ADN à l'aide d'une paire d'amorces incluant l'amorce sens de séquence SEQ ID NO :19 et l'amorce antisens de séquence SEQ ID NO :20 et d'une paire d'amorces incluant l'amorce sens de séquence SEQ ID NO :21 et l'amorce antisens de séquence SEQ ID NO :22, et de deux sondes consistant en les séquences SEQ ID NO :23 et SEQ ID NO :24, dans des conditions permettant la production d'amplicons, et
- (c2) la détection de la présence ou l'absence de borrélies appartenant aux espèces B. hermsii et B. recurrentis, par détection de la présence d'un signal de fluorescence résultant de la formation d'amplicons, et,
- (c3) l'amplification de séquences nucléotidiques cibles dudit ADN à l'aide d'une paire d'amorces incluant l'amorce sens de séquence SEQ ID NO :25 et l'amorce antisens de séquence SEQ ID NO :26 et d'une sonde consistant en la séquence SEQ ID NO :27, dans des conditions permettant la production d'amplicons, et,
- (c4) la détection de la présence ou l'absence de Borrélies appartenant aux espèces B. duttonii ou B. crocidurae par détection de la présence d'un signal de fluorescence résultant de la formation d'amplicons,
(d) l'analyse des résultats des amplifications,
étant entendu que ladite méthode inclut les séquences présentant au moins 90% d'homologie avec les séquences SEQ ID NO : 1 à 4 et 8 à 27.

2. Méthode selon la revendication 1, **caractérisée en ce que** l'étape (a) inclut l'amplification de séquences nucléotidiques codant l'actine de l'hôte à l'aide d'une paire d'amorces incluant l'amorce sens de séquence SEQ ID NO :5 et l'amorce antisens de séquence SEQ ID NO :6 et d'une sonde consistant en la séquences SEQ ID NO :7, ou de séquences présentant au moins 90% d'homologie avec les séquences SEQ ID NO : 5 à 7, dans des conditions permettant la production d'amplicons, puis la détection de la présence ou de l'absence de ladite séquence nucléotidique codant l'actine par détection de la présence d'un signal de fluorescence résultant de la formation d'amplicons.

3. Méthode selon l'une des revendications 1 à 2, **caractérisée en ce que** les étapes (b1) et (b2) comprennent :
- une étape (b1a) consistant en l'amplification de séquences nucléotidiques cibles dudit ADN à l'aide d'une paire d'amorces incluant l'amorce sens de séquence SEQ ID NO :8 et les amorces antisens de séquences SEQ ID NO :9 et SEQ ID NO :10 et deux sondes SEQ ID NO :12 et SEQ ID NO :13,
- (b2a) la détection de la présence ou l'absence de Borrélies appartenant aux espèces B. garinii ou B. afzelii par détection de la présence d'un signal de fluorescence résultant de la formation d'amplicons, et
- une étape (b1b) consistant en l'amplification de séquences nucléotidiques cibles dudit ADN à l'aide d'une paire d'amorces incluant l'amorce sens de séquence SEQ ID NO :8 et les amorces antisens de séquences SEQ ID NO :9 et SEQ ID NO :10 et une sonde SEQ ID NO :11.
- (b2b) la détection de la présence ou l'absence de Borrélies appartenant aux espèces B. burgdorferi sensu stricto par détection de la présence d'un signal de fluorescence résultant de la formation d'amplicons.

4. Méthode selon l'une des revendications 1 à 3, **caractérisée en ce que** l'étape (d) inclut le séquençage des amplicons générés à l'issue des étapes (a), (b)et/ou (c).

5. Méthode selon l'une des revendications 1 à 4, **caractérisée en ce que** les conditions d'amplification réalisées à l'étape (a) et à l'étape (c1) et (c3) sont une dénaturation initiale à 95°C durant 5 minutes puis la mise en œuvre de 45 cycles incluant chacun une dénaturation à 94°C durant 15 sec puis une hybridation et élongation à 58°C durant 45 sec.

6. Méthode selon l'une des revendications 3 à 5, **caractérisée en ce que** les conditions d'amplification réalisées à l'étape (b1a) sont une dénaturation initiale à 95°C durant 5 minutes puis la mise en œuvre de 45 cycles incluant chacun une dénaturation à 94°C durant 15 sec puis une hybridation et élongation à 62°C durant 45 sec.

7. Méthode selon l'une des revendications 1 à 5, **caractérisée en ce que** les conditions d'amplification réalisées à l'étape (b1b) sont une dénaturation initiale à 95°C durant 5 minutes puis la mise en œuvre de 45 cycles incluant chacun une dénaturation à 94°C durant 15 sec puis une hybridation et élongation à 60°C durant 45 sec.

8. Méthode selon l'une des revendications 1 à 6, **caractérisée en ce que** les conditions d'amplification réalisées à l'étape (b3) sont une dénaturation initiale à 95°C durant 5 minutes puis la mise en œuvre de 45 cycles incluant chacun une dénaturation à 94°C durant 15 sec puis une hybridation et élongation à 64°C durant 45 sec.

9. Méthode selon l'une des revendications 1 à 7, **caractérisée en ce que** les sondes de séquence SEQ ID NO : 3, SEQ ID NO : 4, SEQ ID NO :7, SEQ ID NO :11, SEQ ID NO :12, SEQ ID NO :13, SEQ ID NO :17, SEQ ID NO :18, SEQ ID NO :23, SEQ ID NO :24 et SEQ ID NO :27 sont marquées avec une molécule quencher sur l'extrémité 3' et un fluorophore sur l'extrémité 5', la détection d'un signal de fluorescence témoin d'une amplification étant réalisée lors de la dissociation du fluorophore et de la molécule quencher, les sondes utilisées au cours d'une même étape (a), (b1), (b3), (c1), (c3) sont marquées par des fluorochromes différents.

10. Méthode selon l'une des revendications 1 à 8, **caractérisée en ce que** les fluorophores sont choisis parmi 6-FAM, VIC, Cy5, HEX et l'inhibiteur de fluorescence peut être le BlackBerry Quencher ou une molécule MGB associée à un quencher non fluorescent tel que le NFQ.

11. Méthode selon l'une des revendications 1 à 9, **caractérisée en ce que** l'échantillon est du sang, de l'urine, de la salive, un tissu cellulaire.

12. Sonde constituée d'une séquence nucléotidique choisie parmi : SEQ ID NO : 3, SEQ ID NO : 4, SEQ ID NO :7, SEQ ID NO :11, SEQ ID NO :12, SEQ ID NO :13, SEQ ID NO :17, SEQ ID NO :18, SEQ ID NO :23, SEQ ID NO :24 et SEQ ID NO:27, ainsi que leur séquence complémentaire respective.

13. Sonde selon la revendication 12, **caractérisée en ce que** les extrémités 5' des séquences sont marquées par le 6-FAM, le VIC, le CY5, le HEX.

14. Kit pour la détection de la présence de borrélies dans un échantillon humain ou animal et la discrimination et identification des espèces de borrélies, par PCR multiplexes quantitatives, comprenant :
(i) une paire d'amorces incluant l'amorce sens de séquence SEQ ID NO :1 et l'amorce antisens de séquence SEQ ID NO :2 et deux sondes consistant en les séquences SEQ ID NO :3 et SEQ ID NO :4, ou toute séquence présentant une homologie d'au moins 90%, préférentiellement d'au moins 95%, plus préférentiellement d'au moins 98%, avec l'une desdites séquences, ou toute séquence complémentaire,
(ii) des paires d'amorces formées par l'amorce sens de séquence SEQ ID NO :8 et les amorces antisens de séquence SEQ ID NO :9 et SEQ ID NO :10, et trois sondes consistant en les séquences SEQ ID NO :11, SEQ ID NO :12 et SEQ ID NO :13, ou toute séquence présentant une homologie d'au moins 90%, préférentiellement d'au moins 95%, plus préférentiellement d'au moins 98%, avec l'une desdites séquences, ou toute séquence complémentaire,
(iii) des paires d'amorces formées par l'amorce sens de séquence SEQ ID NO :14, l'amorce sens de séquence SEQ ID NO :15 et l'amorce antisens de séquence SEQ ID NO :16, et deux sondes consistant en les séquences SEQ ID NO :17 et SEQ ID NO :18, ou toute séquence présentant une homologie d'au moins 90%, préférentiellement d'au moins 95%, plus préférentiellement d'au moins 98%, avec l'une desdites séquences, ou toute séquence complémentaire,
(iv) une paire d'amorces incluant l'amorce sens de séquence SEQ ID NO :19 et l'amorce antisens de séquence SEQ ID NO :20 et une paire d'amorces incluant l'amorce sens de séquence SEQ ID NO :21 et l'amorce antisens de séquence SEQ ID NO :22, et deux sondes consistant en les séquences SEQ ID NO :23 et SEQ ID NO :24, ou toute séquence présentant une homologie d'au moins 90%, préférentiellement d'au moins 95%, plus préférentiellement d'au moins 98%, avec l'une desdites séquences, ou toute séquence complémentaire,
(v) une paire d'amorces incluant l'amorce sens de séquence SEQ ID NO :25 et l'amorce antisens de séquence SEQ ID NO :26 et une sonde consistant en la séquence SEQ ID NO :27, ou toute séquence présentant une homologie d'au moins 90%, préférentiellement d'au moins 95%, plus préférentiellement d'au moins 98%, avec l'une desdites séquences, ou toute séquence complémentaire,
(vi) ainsi que tous réactifs nécessaires à la réalisation de PCR quantitative multiplexe,
les sondes étant marquées avec une molécule quencher sur l'extrémité 3' et un fluorophore sur l'extrémité 5'.

15. Utilisation d'un kit tel que défini dans la revendication 15 pour la détection de la présence de borrélies dans un échantillon humain ou animal et l'identification des espèces de borrélies, par PCR multiplexes quantitatives.

## Patentansprüche

1. Verfahren zum Nachweis des Vorhandenseins von Borrelien in einer menschlichen oder tierischen Probe und zum Identifizieren von Borrelienspezies durch quantitative Multiplex-PCR, umfassend:
(a) einen Schritt zum Nachweis des Vorhandenseins von Borrelien in der Probe und zum Unterscheiden der Borrelien zwischen der Gruppe, die Lyme-Krankheit verursacht, und der Gruppe, die wiederkehrendes Fieber verursacht, wobei der Schritt besteht aus:
- Extraktion von DNA aus der Probe,
- Amplifikation von Zielnukleotidsequenzen der DNA unter Verwendung eines Primerpaars, das den Sense-Primer der Sequenz SEQ ID NO: 1 und den Antisense-Primer der Sequenz SEQ ID NO: 2 umfasst, und zweier Sonden, bestehend aus den Sequenzen SEQ ID NO: 3 und SEQ ID NO: 4, unter Bedingungen, die die Produktion von Amplikons ermöglichen, und
- Nachweis des Vorhandenseins oder Nichtvorhandenseins von Borrelien der Gruppe, die Lyme-Krankheit verursacht, oder der Gruppe, die wiederkehrendes Fieber verursacht, durch Nachweisen des Vorhandenseins eines Fluoreszenzsignals, das aus der Bildung von Amplikons resultiert, und dann
wenn in Schritt (a) ein Signal nachgewiesen wird, das das Vorhandensein von Borrelien aus der Lyme-Krankheit-Gruppe anzeigt,
(b) einen Schritt zum Nachweis und zur Unterscheidung des Vorhandenseins der Spezies B. burgdorferi sensu stricto, B. garinii oder B. afzelii und der Spezies B. valaisiana oder B. bissettii, in der Probe, wobei der Schritt besteht aus:
- ausgehend von der DNA, die aus dieser Probe extrahiert wurde,
- (b1) Amplifikation von Zielnukleotidsequenzen der DNA unter Verwendung von Primerpaaren, die durch den Sense-Primer der Sequenz SEQ ID NO: 8 und die Antisense-Primer der Sequenzen SEQ ID NO: 9 und SEQ ID NO: 10 gebildet werden, und dreier Sonden, bestehend aus den Sequenzen SEQ ID NO: 11, SEQ ID NO: 12 und SEQ ID NO: 13, unter Bedingungen, die die Produktion von Amplikons ermöglichen, und
- (b2) Nachweis des Vorhandenseins oder Nichtvorhandenseins von Borrelien der Spezies B. burgdorferi sensu stricto, B. garinii oder B. afzelii durch Nachweisen des Vorhandenseins eines Fluoreszenzsignals, das aus der Bildung von Amplikons resultiert, und
- (b3) Amplifikation von Zielnukleotidsequenzen der DNA unter Verwendung von Primerpaaren, die durch den Sense-Primer der Sequenz SEQ ID NO: 14, den Sense-Primer der Sequenz SEQ ID NO: 15 und den Antisense-Primer der Sequenz SEQ ID NO: 16 gebildet werden, und zweier Sonden, bestehend aus den Sequenzen SEQ ID NO: 17 und SEQ ID NO: 18, unter Bedingungen, die die Produktion von Amplikons ermöglichen, und
- (b4) Nachweis des Vorhandenseins oder Nichtvorhandenseins von Borrelien der Spezies B. valaisiana oder B. bissettii durch Nachweisen des Vorhandenseins eines Fluoreszenzsignals, das aus der Bildung von Amplikons resultiert,
oder wenn in Schritt (a) ein Signal nachgewiesen wird, das das Vorhandensein von Borrelien aus der Gruppe mit wiederkehrendem Fieber anzeigt,
(c) einen Schritt zum Nachweis und zur Unterscheidung des Vorhandenseins der Spezies B. hermsii und B. recurrentis, und der Spezies B. duttonii oder B. crocidurae der Gruppe, die wiederkehrende Fieber verursachen, in der Probe, wobei der Schritt besteht aus:
- ausgehend von der DNA, die aus dieser Probe extrahiert wurde,
- (c1) Amplifikation von Zielnukleotidsequenzen der DNA unter Verwendung eines Primerpaars, das den Sense-Primer der Sequenz SEQ ID NO: 19 und den Antisense-Primer der Sequenz SEQ ID NO: 20 umfasst, und eines Primerpaars, das den Sense-Primer der Sequenz SEQ ID NO: 21 und den Antisense-Primer der Sequenz SEQ ID NO: 22 umfasst, und zweier Sonden, die aus den Sequenzen SEQ ID NO: 23 und SEQ ID NO: 24 bestehen, unter Bedingungen, die die Produktion von Amplikons ermöglichen, und
- (c2) Nachweis des Vorhandenseins oder Nichtvorhandenseins von Borrelien der Spezies B. hermsii und B. recurrentis durch Nachweisen des Vorhandenseins eines Fluoreszenzsignals, das aus der Bildung von Amplikons resultiert, und
- (c3) Amplifikation von Zielnukleotidsequenzen der DNA unter Verwendung eines Primerpaars, das den Sense-Primer der Sequenz SEQ ID NO: 25 und den Antisense-Primer der Sequenz SEQ ID NO: 26 umfasst, und einer Sonde, bestehend aus der Sequenz SEQ ID NO: 27, unter Bedingungen, die die Produktion von Amplikons ermöglichen, und
- (c4) Nachweis des Vorhandenseins oder Nichtvorhandenseins von Borrelien der Spezies B. duttonii oder B. crocidurae durch Nachweisen des Vorhandenseins eines Fluoreszenzsignals, das aus der Bildung von Amplikons resultiert,
(d) Analyse der Ergebnisse der Amplifikationen,
wobei es sich versteht, dass das Verfahren die Sequenzen einschließt, die eine Homologie von mindestens 90% mit den Sequenzen SEQ ID NO: 1 bis 4 und 8 bis 27 aufweisen.

2. Verfahren nach Anspruch 1, **dadurch gekennzeichnet, dass** der Schritt (a) die Amplifikation von Nukleotidsequenzen, die Wirts-Aktin codieren, unter Verwendung eines Primerpaars, das den Sense-Primer der Sequenz SEQ ID NO: 5 und den Antisense-Primer der Sequenz SEQ ID NO: 6 umfasst, und einer Sonde, bestehend aus den Sequenzen SEQ ID NO: 7, oder von Sequenzen mit mindestens 90% Homologie zu den Sequenzen SEQ ID NO: 5 bis 7, unter Bedingungen, die die Produktion von Amplikons ermöglichen, und sodann den Nachweis des Vorhandenseins oder Nichtvorhandenseins der Aktin-codierenden Nukleotidsequenz durch Nachweisen des Vorhandenseins eines Fluoreszenzsignals, das aus der Bildung von Amplikons resultiert, umfasst.

3. Verfahren nach einem der Ansprüche 1 bis 2, **dadurch gekennzeichnet, dass** die Schritte (b1) und (b2) umfassen:
- einen Schritt (b1a), der in der Amplifikation von Zielnukleotidsequenzen der DNA unter Verwendung eines Primerpaars, das den Sense-Primer der Sequenz SEQ ID NO: 8 und die Antisense-Primer der Sequenzen SEQ ID NO: 9 und SEQ ID NO: 10 umfasst, und zweier Sonden SEQ ID NO: 12 und SEQ ID NO: 13 besteht,
- (b2a) den Nachweis des Vorhandenseins oder Nichtvorhandenseins von Borrelien, die zu den Spezies B. garinii oder B. afzelii gehören, durch Nachweisen des Vorhandenseins eines Fluoreszenzsignals, das aus der Bildung von Amplikons resultiert, und
- einen Schritt (blb), der in der Amplifikation von Zielnukleotidsequenzen der DNA unter Verwendung eines Primerpaars, das den Sense-Primer der Sequenz SEQ ID NO: 8 und die Antisense-Primer der Sequenzen SEQ ID NO: 9 und SEQ ID NO: 10 umfasst, und einer Sonde SEQ ID NO: 11 besteht.
- (b2b) den Nachweis des Vorhandenseins oder Nichtvorhandenseins von Borrelien der Spezies B. burgdorferi sensu stricto durch Nachweisen des Vorhandenseins eines Fluoreszenzsignals, das aus der Bildung von Amplikons resultiert.

4. Verfahren nach einem der Ansprüche 1 bis 3, **dadurch gekennzeichnet, dass** der Schritt (d) die Sequenzierung von am Ende der Schritte (a), (b) und/oder (c) erzeugten Amplikons umfasst.

5. Verfahren nach einem der Ansprüche 1 bis 4, **dadurch gekennzeichnet, dass** es sich bei den in Schritt (a) und in Schritt (c1) und (c3) durchgeführten Amplifikationsbedingungen um eine anfängliche Denaturierung bei 95°C während 5 Minuten, dann die Durchführung von 45 Zyklen, jeweils umfassend eine Denaturierung bei 94°C während 15 Sekunden, dann eine Hybridisierung und Elongation bei 58°C während 45 Sekunden, handelt.

6. Verfahren nach einem der Ansprüche 3 bis 5, **dadurch gekennzeichnet, dass** es sich bei den in Schritt (b1a) durchgeführten Amplifikationsbedingungen um eine anfängliche Denaturierung bei 95°C während 5 Minuten, dann die Durchführung von 45 Zyklen, jeweils umfassend eine Denaturierung bei 94°C während 15 Sekunden, dann eine Hybridisierung und Elongation bei 62°C während 45 Sekunden, handelt.

7. Verfahren nach einem der Ansprüche 1 bis 5, **dadurch gekennzeichnet, dass** es sich bei den in Schritt (b1b) durchgeführten Amplifikationsbedingungen um eine anfängliche Denaturierung bei 95°C während 5 Minuten, dann die Durchführung von 45 Zyklen, jeweils umfassend eine Denaturierung bei 94°C während 15 Sekunden, dann eine Hybridisierung und Elongation bei 60°C während 45 Sekunden, handelt.

8. Verfahren nach einem der Ansprüche 1 bis 6, **dadurch gekennzeichnet, dass** es sich bei den in Schritt (b3) durchgeführten Amplifikationsbedingungen um eine anfängliche Denaturierung bei 95°C während 5 Minuten, dann die Durchführung von 45 Zyklen, jeweils umfassend eine Denaturierung bei 94°C während 15 Sekunden, dann eine Hybridisierung und Elongation bei 64°C während 45 Sekunden, handelt.

9. Verfahren nach einem der Ansprüche 1 bis 7, **dadurch gekennzeichnet, dass** die Sonden der Sequenz SEQ ID NO: 3, SEQ ID NO: 4, SEQ ID NO: 7, SEQ ID NO: 11, SEQ ID NO: 12, SEQ ID NO: 13, SEQ ID NO: 17, SEQ ID NO: 18, SEQ ID NO: 23, SEQ ID NO: 24 und SEQ ID NO: 27 mit einem Quencher-Molekül am 3'-Ende und einem Fluorophor am 5'-Ende markiert sind, wobei der Nachweis eines Fluoreszenzsignals, das eine Amplifikation anzeigt, während der Dissoziation des Fluorophors und des Quencher-Moleküls durchgeführt wird, wobei die Sonden, die im Verlauf eines und desselben Schrittes (a), (b1), (b3), (cl), (c3) verwendet werden, durch unterschiedliche Fluorochrome markiert sind.

10. Verfahren nach einem der Ansprüche 1 bis 8, **dadurch gekennzeichnet, dass** die Fluorophore ausgewählt sind aus 6-FAM, VIC, Cy5 und HEX, und es sich bei dem Fluoreszenzinhibitor um BlackBerry Quencher oder ein mit einem nicht-fluoreszierenden Quencher wie NFQ assoziiertes MGB-Molekül handeln kann.

11. Verfahren nach einem der Ansprüche 1 bis 9, **dadurch gekennzeichnet, dass** es sich bei der Probe um Blut, Urin, Speichel oder ein Zellgewebe handelt.

12. Sonde bestehend aus einer Nukleotidsequenz, ausgewählt aus: SEQ ID NO: 3, SEQ ID NO: 4, SEQ ID NO: 7, SEQ ID NO: 11, SEQ ID NO: 12, SEQ ID NO: 13, SEQ ID NO: 17, SEQ ID NO: 18, SEQ ID NO: 23, SEQ ID NO: 24 und SEQ ID NO: 27 sowie deren jeweiliger komplementärer Sequenz.

13. Sonde nach Anspruch 12, **dadurch gekennzeichnet, dass** die 5'-Enden der Sequenzen mit 6-FAM, VIC, CY5 oder HEX markiert sind.

14. Kit zum Nachweis des Vorhandenseins von Borrelien in einer menschlichen oder tierischen Probe und zur Unterscheidung und Identifizierung von Borrelienspezies durch quantitative Multiplex-PCR, umfassend:
(i) ein Primerpaar, das den Sense-Primer der Sequenz SEQ ID NO: 1 und den Antisense-Primer der Sequenz SEQ ID NO: 2 umfasst, und zwei Sonden, bestehend aus den Sequenzen SEQ ID NO: 3 und SEQ ID NO: 4, oder jedwede Sequenz mit einer Homologie von mindestens 90%, vorzugsweise mindestens 95%, bevorzugter mindestens 98%, zu einer der genannten Sequenzen oder jedwede komplementäre Sequenz,
(ii) Primerpaare, die durch den Sense-Primer der Sequenz SEQ ID NO: 8 und die Antisense-Primer der Sequenz SEQ ID NO: 9 und SEQ ID NO: 10 gebildet werden, und drei Sonden, bestehend aus den Sequenzen SEQ ID NO: 11, SEQ ID NO: 12 und SEQ ID NO: 13, oder jedwede Sequenz mit einer Homologie von mindestens 90%, vorzugsweise mindestens 95%, bevorzugter mindestens 98%, zu einer der genannten Sequenzen oder jedwede komplementäre Sequenz,
(iii) Primerpaare, die durch den Sense-Primer der Sequenz SEQ ID NO: 14, den Sense-Primer der Sequenz SEQ ID NO: 15 und den Antisense-Primer der Sequenz SEQ ID NO: 16 gebildet werden, und zwei Sonden, bestehend aus den Sequenzen SEQ ID NO: 17 und SEQ ID NO: 18, oder jedwede Sequenz mit einer Homologie von mindestens 90%, vorzugsweise mindestens 95%, bevorzugter mindestens 98%, zu einer der genannten Sequenzen oder jedwede komplementäre Sequenz,
(iv) ein Primerpaar, das den Sense-Primer der Sequenz SEQ ID NO: 19 und den Antisense-Primer der Sequenz SEQ ID NO: 20 umfasst, und ein Primerpaar, das den Sense-Primer der Sequenz SEQ ID NO: 21 und den Antisense-Primer der Sequenz SEQ ID NO: 22 umfasst, und zwei Sonden, bestehend aus den Sequenzen SEQ ID NO: 23 und SEQ ID NO: 24, oder jedwede Sequenz mit einer Homologie von mindestens 90%, vorzugsweise mindestens 95%, bevorzugter mindestens 98%, zu einer der genannten Sequenzen oder jedwede komplementäre Sequenz,
(v) ein Primerpaar, das den Sense-Primer der Sequenz SEQ ID NO: 25 und den Antisense-Primer der Sequenz SEQ ID NO: 26 umfasst, und eine Sonde, bestehend aus der Sequenz SEQ ID NO: 27, oder jedwede Sequenz mit einer Homologie von mindestens 90%, vorzugsweise mindestens 95%, bevorzugter mindestens 98%, zu einer der genannten Sequenzen oder jedwede komplementäre Sequenz,
(vi) sowie alle zur Durchführung der quantitativen Multiplex-PCR erforderlichen Reagenzien,
wobei die Sonden am 3'-Ende mit einem Quencher-Molekül und am 5'-Ende mit einem Fluorophor markiert sind.

15. Verwendung eines Kits, wie in Anspruch 15 definiert, zum Nachweis des Vorhandenseins von Borrelien in einer menschlichen oder tierischen Probe und zur Identifizierung der Borrelienspezies durch quantitative Multiplex-PCR.

## Claims

1. Method for detecting the presence of Borrelia in a human or animal sample and for identifying Borrelia species, by multiplex quantitative PCR, comprising:
(a) a step of detecting the presence of Borrelia in the sample and of distinguishing said Borrelia between the group causing Lyme disease and the group causing relapsing fever, said step consisting of:
- the extraction of DNA from said sample,
- the amplification of target nucleotide sequences of said DNA using a pair of primers including the forward primer of sequence SEQ ID NO: 1 and the reverse primer of sequence SEQ ID NO: 2 and two probes consisting of the sequences SEQ ID NO: 3 and SEQ ID NO: 4, under conditions which allow the production of amplicons, and
- the detection of the presence or absence of Borrelia from the group causing Lyme disease or from the group causing relapsing fever, by detection of the presence of a fluorescence signal resulting from the formation of amplicons, then
if a signal is detected in step (a) indicating the presence of Borrelia from the Lyme disease group,
(b) a step of detecting and distinguishing, in the sample, the presence of the species *B. burgdorferi sensu stricto, B. garinii* or *B. afzelii* and of the species *B. valaisiana* or *B. bissettii,* said step consisting of:
- starting from the DNA extracted from said sample,
- (b1) the amplification of target nucleotide sequences of said DNA using pairs of primers formed by the forward primer of sequence SEQ ID NO: 8 and the reverse primers of sequences SEQ ID NO: 9 and SEQ ID NO: 10 and three probes consisting of the sequences SEQ ID NO: 11, SEQ ID NO: 12 and SEQ ID NO: 13, under conditions which allow the production of amplicons, and
- (b2) the detection of the presence or absence of Borrelia belonging to the species *B. burgdorferi sensu stricto, B. garinii* or *B. afzelii,* by detecting the presence of a fluorescence signal resulting from the formation of amplicons, and
- (b3) the amplification of target nucleotide sequences of said DNA using pairs of primers formed by the forward primer of sequence SEQ ID NO: 14, the forward primer of sequence SEQ ID NO: 15 and the reverse primer of sequence SEQ ID NO: 16, and two probes consisting of the sequences SEQ ID NO: 17 and SEQ ID NO: 18, under conditions which allow the production of amplicons, and
- (b4) the detection of the presence or absence of Borrelia belonging to the species *B. valaisiana* or *B. bissettii,* by detection of the presence of a fluorescence signal resulting from the formation of amplicons,
or, if a signal is detected in step (a) indicating the presence of Borrelia from the relapsing fever group,
(c) a step of detecting and distinguishing, in the sample, the presence of the species *B. hermsii* and *B. recurrentis,* and of the species *B. duttonii* or *B. crocidurae* from the group causing relapsing fever, said step consisting of:
- starting from the DNA extracted from said sample
- (c1) the amplification of target nucleotide sequences of said DNA using a pair of primers including the forward primer of sequence SEQ ID NO: 19 and the reverse primer of sequence SEQ ID NO: 20 and a pair of primers including the forward primer of sequence SEQ ID NO: 21 and the reverse primer of sequence SEQ ID NO: 22, and two probes consisting of the sequences SEQ ID NO: 23 and SEQ ID NO: 24, under conditions which allow the production of amplicons, and
- (c2) the detection of the presence or absence of Borrelia belonging to the species *B. hermsii* and *B. recurrentis,* by detection of the presence of a fluorescence signal resulting from the formation of amplicons, and
- (c3) the amplification of target nucleotide sequences of said DNA using a pair of primers including the forward primer of sequence SEQ ID NO: 25 and the reverse primer of sequence SEQ ID NO: 26 and a probe consisting of the sequence SEQ ID NO: 27, under conditions which allow the production of amplicons, and
- (c4) the detection of the presence or absence of Borrelia belonging to the species *B. duttonii* or *B. crocidurae,* by detection of the presence of a fluorescence signal resulting from the formation of amplicons,
(d) analysing the results of the amplifications,
it being understood that said method includes the sequences having at least 90% homology with the sequences SEQ ID Nos 1 to 4 and 8 to 27.

2. Method according to Claim 1, **characterized in that** step (a) includes the amplification of nucleotide sequences encoding the host's actin using a pair of primers including the forward primer of sequence SEQ ID NO: 5 and the reverse primer of sequence SEQ ID NO: 6 and a probe consisting of the sequence SEQ ID NO: 7, or of sequences having at least 90% homology with the sequences SEQ ID Nos 5 to 7, under conditions which allow the production of amplicons, then the detection of the presence or absence of said nucleotide sequence encoding actin by detection of the presence of a fluorescence signal resulting from the formation of amplicons.

3. Method according to either of Claims 1 and 2, **characterized in that** steps (b1) and (b2) comprise:
- a step (b1a) consisting of the amplification of target nucleotide sequences of said DNA using a pair of primers including the forward primer of sequence SEQ ID NO: 8 and the reverse primers of sequences SEQ ID NO: 9 and SEQ ID NO: 10, and two probes SEQ ID NO: 12 and SEQ ID NO: 13,
- (b2a) the detection of the presence or absence of Borrelia belonging to the species *B. garinii* or *B. afzelii,* by detection of the presence of a fluorescence signal resulting from the formation of amplicons, and
- a step (b1b) consisting of the amplification of target nucleotide sequences of said DNA using a pair of primers including the forward primer of sequence SEQ ID NO: 8 and the reverse primers of sequences SEQ ID NO: 9 and SEQ ID NO: 10, and a probe SEQ ID NO: 11,
- (b2b) the detection of the presence or absence of Borrelia belonging to the species *B. burgdorferi sensu stricto,* by detection of the presence of a fluorescence signal resulting from the formation of amplicons.

4. Method according to one of Claims 1 to 3, **characterized in that** step (d) includes the sequencing of the amplicons generated at the outcome of steps (a), (b) and/or (c).

5. Method according to one of Claims 1 to 4, **characterized in that** the amplification conditions carried out in step (a) and in steps (c1) and (c3) are an initial denaturation at 95°C for 5 minutes, then the performing of 45 cycles, each including a denaturation at 94°C for 15 sec, then a hybridization and elongation at 58°C for 45 sec.

6. Method according to one of Claims 3 to 5, **characterized in that** the amplification conditions carried out in step (b1a) are an initial denaturation at 95°C for 5 minutes, then the performing of 45 cycles, each including a denaturation at 94°C for 15 sec, then a hybridization and elongation at 62°C for 45 sec.

7. Method according to one of Claims 1 to 5, **characterized in that** the amplification conditions carried out in step (b1b) are an initial denaturation at 95°C for 5 minutes, then the performing of 45 cycles, each including a denaturation at 94°C for 15 sec, then a hybridization and elongation at 60°C for 45 sec.

8. Method according to one of Claims 1 to 6, **characterized in that** the amplification conditions carried out in step (b3) are an initial denaturation at 95°C for 5 minutes, then the performing of 45 cycles, each including a denaturation at 94°C for 15 sec, then a hybridization and elongation at 64°C for 45 sec.

9. Method according to one of Claims 1 to 7, **characterized in that** the probes of sequence SEQ ID NO: 3, SEQ ID NO: 4, SEQ ID NO: 7, SEQ ID NO: 11, SEQ ID NO: 12, SEQ ID NO: 13, SEQ ID NO: 17, SEQ ID NO: 18, SEQ ID NO: 23, SEQ ID NO: 24 and SEQ ID NO: 27 are labelled with a quencher molecule on the 3' end and a fluorophore on the 5' end, the detection of a fluorescence signal attesting to an amplification being carried out during the dissociation of the fluorophore and the quencher molecule; the probes used during one and the same step (a), (b1), (b3), (c1), (c3) are labelled with different fluorochromes.

10. Method according to one of Claims 1 to 8, **characterized in that** the fluorophores are chosen from 6-FAM, VIC, Cy5 and HEX, and the fluorescence inhibitor may be the BlackBerry Quencher or an MGB molecule linked to a non-fluorescent quencher such as NFQ.

11. Method according to one of Claims 1 to 9, **characterized in that** the sample is blood, urine, saliva or a cell tissue.

12. Probe consisting of a nucleotide sequence chosen from: SEQ ID NO: 3, SEQ ID NO: 4, SEQ ID NO: 7, SEQ ID NO: 11, SEQ ID NO: 12, SEQ ID NO: 13, SEQ ID NO: 17, SEQ ID NO: 18, SEQ ID NO: 23, SEQ ID NO: 24 and SEQ ID NO: 27, and also the respective sequence complementary thereto.

13. Probe according to Claim 12, **characterized in that** the 5' ends of the sequences are labelled with 6-FAM, VIC, Cy5, HEX.

14. Kit for detecting the presence of Borrelia in a human or animal sample and distinguishing and identifying Borrelia species, by multiplex quantitative PCR, comprising:
(i) a pair of primers including the forward primer of sequence SEQ ID NO: 1 and the reverse primer of sequence SEQ ID NO: 2 and two probes consisting of the sequences SEQ ID NO: 3 and SEQ ID NO: 4, or any sequence having at least 90%, preferentially at least 95%, more preferentially at least 98%, homology with one of said sequences, or any complementary sequence,
(ii) pairs of primers formed by the forward primer of sequence SEQ ID NO: 8 and the reverse primers of sequence SEQ ID NO: 9 and SEQ ID NO: 10, and three probes consisting of the sequences SEQ ID NO: 11, SEQ ID NO: 12 and SEQ ID NO: 13, or any sequence having at least 90%, preferentially at least 95%, more preferentially at least 98%, homology with one of said sequences, or any complementary sequence,
(iii) pairs of primers formed by the forward primer of sequence SEQ ID NO: 14, the forward primer of sequence SEQ ID NO: 15 and the reverse primer of sequence SEQ ID NO: 16, and two probes consisting of the sequences SEQ ID NO: 17 and SEQ ID NO: 18, or any sequence having at least 90%, preferentially at least 95%, more preferentially at least 98%, homology with one of said sequences, or any complementary sequence,
(iv) a pair of primers including the forward primer of sequence SEQ ID NO: 19 and the reverse primer of sequence SEQ ID NO: 20 and a pair of primers including the forward primer of sequence SEQ ID NO: 21 and the reverse primer of sequence SEQ ID NO: 22, and two probes consisting of the sequences SEQ ID NO: 23 and SEQ ID NO: 24, or any sequence having at least 90%, preferentially at least 95%, more preferentially at least 98%, homology with one of said sequences, or any complementary sequence,
(v) a pair of primers including the forward primer of sequence SEQ ID NO: 25 and the reverse primer of sequence SEQ ID NO: 26 and a probe consisting of the sequence SEQ ID NO: 27, or any sequence having at least 90%, preferentially at least 95%, more preferentially at least 98%, homology with one of said sequences, or any complementary sequence,
(vi) and also any reagents required for carrying out multiplex quantitative PCR,
the probes being labelled with a quencher molecule on the 3' end and a fluorophore on the 5' end.

15. Use of a kit as defined in Claim 15, for detecting the presence of Borrelia in a human or animal sample and identifying Borrelia species, by multiplex quantitative PCR.
